# EUROPEAN PATENT APPLICATION

(11) **EP 4 685 236 A1**
(43) Date of publication of application: **28.01.2026**
(21) Application number: 24774945.0
(22) Date of filing: 21.03.2024
(51) Int. Cl.: C12N 15/62, C07K 14/115, C07K 14/435, C07K 19/00, C12N 5/10, C12N 15/12, C12N 15/45, C12N 15/86, C12P 21/02

(54) **TEMPERATURE-SENSITIVE NEGATIVE-STRAND RNA VIRUS OR VIRAL VECTOR AND RNA GENOME THEREOF**

(30) Priority: 22.03.2023 JP 2023045475
(71) Applicant: Repli-Tech Co., Ltd., Tokyo 150-0012 (JP)
(72) Inventor: SAEKI, Koichi, Tokyo 150-0012 (JP)
(74) Representative: Hoffmann Eitle
(86) International application number: PCT/JP2024/010883
(87) International publication number: WO 2024/195811

(57) **Abstract**

The present invention provides a temperature-sensitive negative-strand RNA virus or viral vector, and its RNA genome. According to the present invention, there is provided a negative-strand RNA virus or viral vector in which at least one of the viral proteins encoded by the genome (preferably the P protein or a temperature-sensitive P protein) is in the form of a fusion protein with a bromotag.

## Description

### Technical Field

The present invention relates to a temperature-sensitive negative-strand RNA virus or virus vector and an RNA genome thereof.

### Background Art

Negative-strand RNA viruses or virus vectors such as Sendai virus are known as cytoplasmic RNA virus vectors, and are believed to be advantageous in regenerative medicine and in vivo applications because they are less likely to damage the nucleus. In addition, negative-strand RNA viruses or virus vectors such as Sendai virus may sometimes exhibit a high gene transfer efficiency and/or gene expression efficiency both in vivo and in vitro and are believed to be useful for gene transfer and/or gene expression purposes, and their development is being actively pursued.

In order to increase the safety of the use of viruses or virus vectors, there is a need for techniques to eliminate, from a cell, viruses or virus vectors with which the cell has been infected (for example, techniques to allow the viruses or virus vectors to decrease in amount or disappear). Temperature-sensitive mutants have been constructed as a technique to eliminate viruses or virus vectors. Specifically, those containing the L511F mutation in the P protein or those having the D433A/R434A/K437A mutations in the P protein have been proposed (Patent Literatures 1 to 4). In addition, techniques have been proposed to introduce N1197S/L1558I/K1796E into the L protein to increase temperature sensitivity (Patent Literatures 3 and 4).

### Citation List

### Patent Literature

Patent Literature 1: International Publication No. WO 2012/029770
Patent Literature 2: International Publication No. WO 2010/008054
Patent Literature 3: International Publication No. WO 2017/082174
Patent Literature 4: International Publication No. WO 2003/025570

### Non Patent Literature

Non Patent Literature 1: PNAS, 108 (34): 14234 - 14239, 2011

### Summary of Invention

According to the present invention, a temperature-sensitive negative-strand RNA virus or virus vector and an RNA genome thereof are provided. According to the present invention, it is possible to culture a virus- or virus vector-infected cell while maintaining the infection and to thereafter eliminate (reduce) the virus or virus vector, depending on the culture temperature conditions.

According to the present invention, the following inventions are provided.
[1] An RNA genome of a negative-strand RNA virus or virus vector, wherein at least one of the viral proteins encoded by the genome (preferably the P protein or a temperature-sensitive P protein) is in the form of a fusion protein with a bromotag.
[2] The RNA genome of the negative-strand RNA virus or viral vector according to [1],
   wherein the temperature-sensitive P protein encoded by the virus genome has a substitution mutation(s) in an amino acid(s) of the P protein corresponding to one or more or all of D433, R434, and K437, the substitution mutation(s) comprising a mutation to any of G, T, and S, and optionally further has a substitution mutation in an amino acid of the P protein corresponding to L511, the substitution mutation in L511 being a mutation to Y or F, or wherein the temperature-sensitive P protein encoded by the virus genome has substitution mutations in the amino acids of the P protein corresponding to one or more or all of D433, R434, and K437, and each of the substitution mutations comprises a mutation to any of G, T, and S.
[3] The RNA genome of a negative-strand RNA virus or virus vector according to [2], wherein each of the substitution mutations is a mutation to T or S.
[4] The RNA genome of a negative-strand RNA virus or virus vector according to [3], wherein each of the substitution mutations is a mutation to T.
[5] The RNA genome of a negative-strand RNA virus or virus vector according to [3], wherein each of the substitution mutations is a mutation to S.
[6] The RNA genome of a negative-strand RNA virus or virus vector according to any of [1] to [3], wherein the temperature-sensitive P protein encoded by the virus genome:
   (1S) has amino acid mutations corresponding to D433S, R434A, K437A and L511F;
   (2S) has amino acid mutations corresponding to D433S, R434A, K437A and L511Y;
   (3S) has amino acid mutations corresponding to D433A, R434S, K437A and L511F;
   (4S) has amino acid mutations corresponding to D433A, R434S, K437A and L511Y;
   (5S) has amino acid mutations corresponding to D433A, R434A, K437S and L511F;
   (6S) has amino acid mutations corresponding to D433A, R434A, K437S and L511Y;
   (7S) has amino acid mutations corresponding to D433S, R434S, K437A and L511F;
   (8S) has amino acid mutations corresponding to D433S, R434S, K437A and L511Y;
   (9S) has amino acid mutations corresponding to D433S, R434A, K437S and L511F;
   (10S) has amino acid mutations corresponding to D433S, R434A, K437S and L511Y;
   (11S) has amino acid mutations corresponding to D433A, R434S, K437S and L511F;
   (12S) has amino acid mutations corresponding to D433A, R434S, K437S and L511Y;
   (13S) has amino acid mutations corresponding to D433S, R434S, K437S and L511F;
   (14S) has amino acid mutations corresponding to D433S, R434S, K437S and L511Y;
   (1T) has amino acid mutations corresponding to D433T, R434A, K437A and L511F;
   (2T) has amino acid mutations corresponding to D433T, R434A, K437A and L511Y;
   (3T) has amino acid mutations corresponding to D433A, R434T, K437A and L511F;
   (4T) has amino acid mutations corresponding to D433A, R434T, K437A and L511Y;
   (5T) has amino acid mutations corresponding to D433A, R434A, K437T and L511F;
   (6T) has amino acid mutations corresponding to D433A, R434A, K437T and L511Y;
   (7T) has amino acid mutations corresponding to D433T, R434T, K437A and L511F;
   (8T) has amino acid mutations corresponding to D433T, R434T, K437A and L511Y;
   (9T) has amino acid mutations corresponding to D433T, R434A, K437T and L511F;
   (10T) has amino acid mutations corresponding to D433T, R434A, K437T and L511Y;
   (11T) has amino acid mutations corresponding to D433A, R434T, K437T and L511F;
   (12T) has amino acid mutations corresponding to D433A, R434T, K437T and L511Y;
   (13T) has amino acid mutations corresponding to D433T, R434T, K437T and L511F;
   (14T) has amino acid mutations corresponding to D433T, R434T, K437T and L511Y;
   (1ST) has amino acid mutations corresponding to D433S, R434T, K437S and L511F; or
   (2ST) has amino acid mutations corresponding to D433S, R434T, K437S and L511Y.
[7] The RNA genome of a negative-strand RNA virus or virus vector according to any of [1] to [6] above, wherein a large protein (L protein) has an amino acid mutation(s) corresponding to one or both of L1361C and L1558I.
[8] The RNA genome of a negative-strand RNA virus or virus vector according to any of [1] to [7] above, having at least one exogenous gene of interest loaded thereon.
[9] The RNA genome of a negative-strand RNA virus or virus vector according to any of [1] to [8] above, which is a Sendai virus or Sendai virus vector.
[10] A nucleic acid encoding the RNA genome of a negative-strand RNA virus or virus vector according to any of [1] to [9] above {preferably DNA, more preferably an expression vector for the genome RNA (particularly plasmid or the like)}.
[11A] A negative-strand RNA virus or viral vector comprising the genomic RNA of any one of [1] to [9], wherein preferably at least one of the viral proteins encoded by the genome (preferably the P protein or a temperature-sensitive P protein) is in the form of a fusion protein with a bromotag, and/or at least one of the viral proteins constituting the virus or viral vector (preferably the P protein or a temperature-sensitive P protein) is in the form of a fusion protein with a bromotag.
[11B] A negative-strand RNA virus or viral vector comprising the genomic RNA of any one of [1] to [9], wherein preferably at least one of the viral proteins encoded by the genome (preferably the P protein or a temperature-sensitive P protein) is in the form of a fusion protein with a bromotag.
[11C] A negative-strand RNA virus or viral vector comprising the genomic RNA of any one of [1] to [9], wherein preferably at least one of the viral proteins constituting the virus or viral vector (preferably the P protein or a temperature-sensitive P protein) is in the form of a fusion protein with a bromotag.
[11D] A negative-strand RNA virus or viral vector comprising the genomic RNA of any one of [1] to [9], wherein preferably at least one of the viral proteins encoded by the genome (preferably the P protein or a temperature-sensitive P protein) is in the form of a fusion protein with a bromotag, and at least one of the viral proteins constituting the virus or viral vector (preferably the P protein or a temperature-sensitive P protein) is in the form of a fusion protein with a bromotag.
[11E] A negative-strand RNA virus or viral vector wherein at least one of the viral proteins encoded by the genome (preferably the P protein or a temperature-sensitive P protein) is in the form of a fusion protein with a bromotag, and/or at least one of the viral proteins constituting the virus or viral vector (preferably the P protein or a temperature-sensitive P protein) is in the form of a fusion protein with a bromotag.

Hereinafter, [11A] to [11E] are collectively referred to as [11].
[12] The negative-strand RNA virus or virus vector according to [11], comprising either a P protein weaker in temperature sensitivity than the temperature-sensitive P protein encoded on the genome or a P protein having no temperature sensitivity.
[13] The negative-strand RNA virus or virus vector according to [11] or [12], wherein when the L protein encoded on the virus genome is temperature-sensitive, the virus or virus vector comprises an L protein weaker in temperature sensitivity than the L protein encoded on the genome or an L protein having no temperature sensitivity.
[14] A composition comprising the negative-strand RNA virus or virus vector according to any of [11] to [13].
[15] A method for expressing gene of interest in a cell, a method for producing a cell expressing a gene of interest, or a method for culturing a cell, comprising:
   infecting the cell with the negative-strand RNA virus or virus vector according to any of [11] to [13] and then culturing the cell under the first temperature conditions to express the gene of interest in the cell; and
   thereafter further culturing the cell under the second temperature conditions, thereby partly or fully eliminating the negative-strand RNA virus or virus vector from the cell.
[16] A method for expressing a gene of interest in a cell, comprising:
   infecting the cell with the negative-strand RNA virus or virus vector according to any of [11] to [13] above and then culturing the cell under the first temperature conditions to express the gene of interest in the cell; and
   thereafter further culturing under the second temperature conditions, thereby partly or fully eliminating the negative-strand RNA virus or virus vector.
[17] A method for producing a cell expressing a gene of interest, comprising:
   infecting the cell with the negative-strand RNA virus or virus vector according to any of [11] to [13] above and then culturing the cell under the first temperature conditions to express the gene of interest in the cell; and
   thereafter further culturing under the second temperature conditions, thereby partly or fully eliminating the negative-strand RNA virus or virus vector.
[18] A method for culturing a cell, comprising:
   infecting the cell with the negative-strand RNA virus or virus vector according to any of [11] to [13] above and then culturing the cell under the first temperature conditions to express the gene of interest in the cell; and
   thereafter further culturing under the second temperature conditions, thereby partly or fully eliminating the negative-strand RNA virus or virus vector.
[19] A method for expressing a gene of interest in a cell, comprising:
   infecting the cell with the negative-strand RNA virus or virus vector according to any of [11] to [13] above and then culturing the cell under the first temperature conditions to express the gene of interest in the cell.
[20] A method for producing a cell expressing the gene of interest, comprising:
   infecting the cell with the negative-strand RNA virus or virus vector according to any of [11] to [13] above and then culturing the cell under the first temperature conditions to express the gene of interest in the cell.
[21] A method for culturing a cell, comprising:
   infecting the cell with the negative-strand RNA virus or virus vector according to any of [11] to [13] above and then culturing the cell under the first temperature conditions to express the gene of interest in the cell.
[22] A method for expressing a gene of interest in a cell, comprising:
   infecting the cell with the negative-strand RNA virus or virus vector according to any of [11] to [13] above and then culturing the cell under the second temperature conditions to express the gene of interest in the cell (preferably while eliminating the virus or virus vector from the cell).
[23] A method for producing a cell expressing a gene of interest, comprising:
   infecting the cell with the negative-strand RNA virus or virus vector according to any of [11] to [13] above and then culturing the cell under the second temperature conditions to express the gene of interest in the cell (preferably while eliminating the virus or virus vector from the cell).
[24] A method for culturing a cell, comprising:
   infecting the cell with the negative-strand RNA virus or virus vector according to any of [11] to [13] above and then culturing the cell under the second temperature conditions to express the gene of interest in the cell (preferably while eliminating the virus or virus vector from the cell).
[25] The invention according to any of the above, wherein the negative-strand RNA virus or virus vector is Sendai virus.
[26] The invention according to any of the above, wherein the negative-strand RNA virus or virus vector has an RNA genome and all of an N protein, a P protein or a temperature-sensitive P protein, an M protein, an F protein, an HN protein, and an L protein {wherein the RNA genome preferably has no gene encoding a functional F protein}.
[101] A degradation-inducible protein, preferably a degradation-inducible protein derived from FKBP12, having an amino acid sequence having amino acid mutations corresponding to one or more, two or more, three or more, four or more, five or more, six or more, seven or more, eight or more, nine or more, ten or more, or all amino acid mutations selected from the group consisting of 4I, 18K, 36L, 50I, 57K, 59F, 63A, 68L, 90V, 98I, and 105R, relative to the amino acid sequence set forth in SEQ ID NO: 18.
[102] A degradation-inducible protein, preferably a degradation-inducible protein derived from FKBP12, having an amino acid sequence having amino acid mutations corresponding to one or more, two or more, three or more, four or more, five or more, six or more, seven or more, eight or more, nine or more, or all amino acid mutations selected from the group consisting of 4I, 18K, 50I, 57K, 59F, 63A, 68L, 90V, 98I, and 105R, and an amino acid mutation corresponding to 36L (and preferably 90V and/or 106P), relative to the amino acid sequence set forth in SEQ ID NO: 18.
[103] The degradation-inducible protein according to [101] or [102] above, having amino acid mutations corresponding to all of 4I, 18K, 36L, 50I, 57K, 59F, 63A, 68L, 90V, 98I, and 105R, relative to the amino acid sequence set forth in SEQ ID NO: 18.
[104] A degradation-inducible fusion protein comprising a protein of interest and the degradation-inducible protein according to any of [101] to [103] above.
[105] A nucleic acid (such as DNA or RNA) encoding the protein according to any of [101] to [104] above.
[106] An antisense oligo to a nucleic acid encoding the protein according to any of [101] to [103] above {preferably containing an antisense moiety to a nucleic acid encoding one or more of the above amino acid mutations}.
[107] An antibody binding to the protein according to any of [101] to [103] above.
[108] An antibody binding to the protein according to any of [101] to [103] above with a stronger binding affinity than to the protein having the amino acid sequence set forth in SEQ ID NO: 18.
[109] An antibody {preferably a monoclonal antibody or polyclonal antibody that has been optionally isolated} binding to the protein according to any of [101] to [103] above with a binding affinity that is two times or more, three times or more, four times or more, five times or more, six times or more, seven times or more, eight times or more, nine times or more, ten times or more, 100 times or more, 1,000 times or more, or 10,000 times or more stronger than to the protein having the amino acid sequence set forth in SEQ ID NO: 18.
[110] An animal cell comprising any of the above proteins.
[111] An animal cell comprising any of the above nucleic acids.
[112] A negative-strand RNA virus or vector comprising any of the above proteins.
[113] A negative-strand RNA virus or vector comprising any of the above nucleic acids.
[114] A negative-strand RNA genome of the virus or vector according to [113] above comprising any of the above nucleic acids.
[115] A nucleic acid encoding the genome according to [114] above, or a vector comprising the nucleic acid.
[131] An M protein having the amino acid sequence set forth in SEQ ID NO: 20.
[132] A nucleic acid encoding the M protein according to [131] above.
[133] An HN protein comprising the amino acid sequence set forth in SEQ ID NO: 21.
[134] A nucleic acid encoding the HN protein according to [133] above.
[135] An animal cell comprising the M protein according to [131] above and/or the HN protein according to [133] above.
[136] An animal cell comprising the M protein according to [131] above and the HN protein according to [133] above.
[137] A negative-strand RNA virus or vector comprising the M protein according to [131] above and/or the HN protein according to [133] above.
[138] A nucleic acid encoding the negative-strand RNA virus or vector according to [137] above, or a vector comprising the nucleic acid.
[139] An animal cell comprising the nucleic acid according to [132] above and/or the nucleic acid according to [134] above.
[140] A genome of a negative-strand RNA virus or vector (a negative-strand RNA genome) comprising the nucleic acid according to [132] above and/or the nucleic acid according to [134] above.
[141] A negative-strand RNA virus or virus vector comprising the genome according to [140] above.
[201] A negative-strand RNA virus or vector, comprising one or more proteins selected from the group consisting of N protein, P protein or temperature-sensitive P protein, M protein, F protein, HN protein, and L protein, to which a peptide having the amino acid sequence of SEQ ID NO: 29 is added.
[202] A negative-strand RNA virus or vector, comprising a nucleic acid encoding one or more proteins selected from the group consisting of N protein, P protein or temperature-sensitive P protein, M protein, F protein, HN protein, and L protein, to which a peptide having the amino acid sequence of SEQ ID NO: 29 is added.
[203] The negative-strand RNA virus or vector according to [201] or [202], further comprising a nucleic acid encoding a target protein to which a peptide having the amino acid sequence of SEQ ID NO: 29 is added.
[204] A genome (negative-strand RNA genome) of the negative-strand RNA virus or vector according to any one of [201] to [203].
[205] A nucleic acid encoding the genome according to [204] .
[206] A gene expression vector comprising the nucleic acid according to [205].
[207] A cell (e.g., an animal cell, e.g., a host cell or a packaging cell) comprising the negative-strand RNA virus or vector according to any one of [201] to [203], the genome according to [204], the nucleic acid according to [205], or the gene expression vector according to [206] .
[251] A method for producing a negative-strand RNA virus or vector, comprising introducing into a packaging cell a nucleic acid encoding the negative-strand RNA virus or vector according to any one of [201] to [203], thereby obtaining the negative-strand RNA virus or vector.
[252] A method for treating a cell infected with the negative-strand RNA virus or vector according to any one of [201] to [203], comprising contacting the cell with an effective amount of the peptide and a binding molecule that binds to ubiquitin E3 ligase, thereby removing part or all of the negative-strand RNA virus or vector from the cell.
[253] The method according to [252], wherein the binding molecule is (S)-13-((2S,4R)-4-hydroxy-2-(((4-(4-methylthiazol-5-yl)benzyl)carbamoyl)pyrrolidin-1-carbonyl))-14,14-dimethyl-11-oxo-3,6,9-trioxa-12-azapentadecyl (R)-2-(((S)-4-(4-chlorophenyl)-2,3,9-trimethyl-6H-thieno[3,2-f][1,2,4]triazolo[4,3-a][1,4]diazepin-6-yl)butanoate.
[254] The method according to [252] or [253], carried out under temperature conditions of 37°C to 38.5°C.
[255] The method according to any one of [252] to [254], carried out in vitro.
[256] The method according to any one of [252] to [254], carried out in vivo.
[257] A binding molecule that binds to the peptide and ubiquitin E3 ligase, or a composition comprising the binding molecule, for use in the method according to [256] .
[258] A binding molecule that binds to the peptide and ubiquitin E3 ligase, or a composition comprising the binding molecule, for use in removing the negative-strand RNA virus or vector from the body of a subject having the negative-strand RNA virus or vector according to any one of [201] to [203].
[259] The binding molecule or composition comprising the binding molecule according to [257] or [258], wherein the binding molecule is (S)-13-((2S,4R)-4-hydroxy-2-(((4-(4-methylthiazol-5-yl)benzyl)carbamoyl)pyrrolidin-1-carbonyl))-14,14-dimethyl-11-oxo-3,6,9-trioxa-12-azapentadecyl (R)-2-(((S)-4-(4-chlorophenyl)-2,3,9-trimethyl-6H-thieno[3,2-f][1,2,4]triazolo[4,3-a][1,4]diazepin-6-yl)butanoate.

### Brief Description of Drawings

[Figure 1A] Figure 1A is a fluorescence micrograph of cells infected with each of various temperature-sensitive mutants of the P protein, showing the temperature sensitivity of the temperature-sensitive mutants.
[Figure 1B] Figure 1B shows the relative fluorescence intensity from cells infected with each of various temperature-sensitive mutants of the P protein, showing the temperature sensitivity of the temperature-sensitive mutants.
[Figure 2] Figure 2 shows the relative fluorescence intensity from cells infected with each of various temperature-sensitive mutants of the P protein, showing the temperature sensitivity of the temperature-sensitive mutants.
[Figure 3] Figure 3 shows the relative fluorescence intensity from cells infected with each of various temperature-sensitive mutants of the P protein, showing the temperature sensitivity of the temperature-sensitive mutants.
[Figure 4] Figure 4 shows the relative fluorescence intensity from cells infected with each of various temperature-sensitive mutants having mutations in the P protein and the L protein, showing the temperature sensitivity of the temperature-sensitive mutants.
[Figure 5A] Figure 5A shows the relative fluorescence intensity from cells infected with each of various temperature-sensitive mutants having mutations in the P protein and the L protein, showing the temperature sensitivity of the temperature-sensitive mutants.
[Figure 5B] Figure 5B shows the relative fluorescence intensity from cells infected with each of various temperature-sensitive mutants having mutations in the P protein and the L protein obtained from cells packaging various mutants having Lm1558I, showing the temperature sensitivity of the temperature-sensitive mutants.
[Figure 6] Figure 6 is a graph showing that proliferation of cells into which a cell proliferation factor was introduced by temperature-sensitive vectors is promoted.
[Figure 7A] Figure 7A is a fluorescence micrograph showing the expression of a pluripotent marker, NANOG in cells into which a cell reprogramming factor was introduced by a temperature-sensitive vector.
[Figure 7B] Figure 7B shows a micrograph image of a fibroblast having a reprogramming factor introduced thereinto 21 and 28 days after the introduction. The fibroblast was cultured at 37°C. GFP (mEmerald) has been encoded by a SeV genome, and the GFP intensity shows the amount of the SeV genome in the fibroblast.
[Figure 8] Figure 8 shows the effect of stabilizing EmGFP by a DDrs tag.
[Figure 9A] Figure 9A shows the ability of DDm, a mutant of a degradation-inducing protein DD, to eliminate a temperature-sensitive vector from cells in the absence of a degradation-promoting factor (dTAG-13).
[Figure 9B] Figure 9B shows the ability of DDm, a mutant of a degradation-inducing protein DD, to eliminate a temperature-sensitive vector from cells in the presence of a degradation-promoting factor (dTAG-13).
[Figure 10] Figure 10 shows the influences of introducing a mutation into an M protein and a mutation into an HN protein on the cytotoxicity of infection with a virus vector. The ordinate axis represents the fluorescence intensity of EmGFP. EmGFP has been encoded by an SeV genome. In the present experiment, the control (Control) is accompanied by cell detachment and a decrease in fluorescence-positive cells, and the intensity of EmGFP reflects the number of viable cells.
[Figure 11A] Figure 11A shows the structure of an F-protein introducing vector for preparing a packaging cell.
[Figure 11B] Figure 11B shows the production of an SeV vector (i.e., successful functional introduction of an F protein) in cells (an LLC-MK2 cell and a Vero cell) into which the vector in Figure 11A and SeV expressing a nucleic acid encoding Cre recombinase has been introduced, and elimination of the produced SeV vector from the cells using the temperature sensitivity.
[Figure 12] Figure 12 shows the alignment of the domain of FKBP12 of each animal species corresponding to a destabilized domain (DD) derived from human FKBP12.
[Figure 13] Figure 13 shows the clearance ability in the presence of PROTAC of a temperature-sensitive vector having a P protein to which a bromotag has been added.
[Figure 14] Figure 14 shows the clearance ability in the presence of PROTAC of a temperature-sensitive vector having a P protein to which a bromotag has been added.

### Description of Inventions

As used herein, the "negative-strand RNA virus vector" refers to a recombinant virus obtained by modifying a virus that has a negative-strand RNA as its genome (that is, a negative-strand RNA virus) for transferring a gene of interest. Examples of the negative-strand RNA virus include the family Orthomyxoviridae (orthomyxoviruses such as influenza virus), the family Paramyxoviridae (paramyxoviruses such as the genus Morbillivirus), the family Rhabdoviridae (rhabdoviruses such as rabies virus), the family Filoviridae (filoviruses such as Ebola virus and Marburg virus) and the family Bunyaviridae (bunyaviruses such as hantavirus). Examples of the paramyxovirus include viruses of the subfamily Orthoparamyxovirinae. Examples of the viruses of the subfamily Orthoparamyxovirinae include viruses of the genus Respirovirus such as a Sendai virus. In all embodiment, the negative-strand RNA virus and the negative-strand RNA virus vector are preferably Sendai virus (also referred to as murine parainfluenza type 1 virus) or a Sendai virus vector.

As used herein, the "packaging cell" is a cell that produces a virus vector. Generally, from the viewpoint of improvement in safety, a virus genome of a virus vector is engineered so that factors responsible for one or more functions selected from the group consisting of proliferation, replication, and spread of the virus vector (including infection to other cells) are disrupted and the virus vector cannot grow, replicate or spread after a cell infection. However, when producing the virus vector, the virus vector is produced while complementing the packaging cell with the disrupted factors in order to enable proliferation, replication, and spread of the virus vector. For this reason, the packaging cells are complemented with the disrupted viral factors in order to cause the packaging cell to produce a virus vector, and the packaging cells are caused to express some of the disrupted viral factors so that the virus production level is restored. The packaging cell may stably possess such factors in the genome or may transiently possess such factors. In either case, during virus production, the viral factors to be complemented are supplied intracellularly to the packaging cell. As an example, a Sendai virus vector is classically obtained by producing a Sendai virus having a genome deficient in an F gene using a packaging cell that supplies the F gene. The F gene to be supplied is activated in the presence of trypsin, but a type of F gene (F5R) that is activated by furin which is ubiquitous in cells, has also been developed, making virus production more convenient (see, for example, WO 2005/071085A). In recent years, techniques for producing a Sendai virus from cDNA have been developed. For example, vectors, having the strain Z, an attenuated strain as a basic structure, which are designed to further increase safety for medical applications to humans have been constructed. For example, a technique has been developed to delete any one or more of the M, F, and HN genes from a virus genome, thereby causing a loss of viral spread. For example, an F gene-deleted virus genome is preferably used. The virus genome is operably linked to a regulatory sequence (such as a T7 promoter), and the regulatory sequence can drive the production of the virus genome. Thereby, a Sendai virus genome can be produced from cDNA in the packaging cell. When using the T7 promoter as a first regulatory sequence, a T7 RNA polymerase can be supplied, for example, by a helper virus, such as vaccinia virus. N, P, F, and L operably linked to a regulatory sequence that drives transcription by an RNA polymerase (such as pol II) can be expressed in the packaging cell, thereby supplying a component of a virus particle to form the virus particle in the packaging cell. As the packaging cell, for example, a monkey kidney-derived LLC-MK2 cell is used. This results in a virus particle that can infect a cell once, but cannot spread to other cells thereafter. The virus particle can be used after concentrated and/or purified as necessary. When a foreign gene is integrated into a virus, a regulatory sequence unique to the virus is introduced as necessary to enable transcription by an RNA-dependent RNA polymerase.

According to the present invention, a method for producing a negative-strand RNA virus or a negative-strand RNA virus vector is provided. Examples of the negative-strand RNA virus include the family Orthomyxoviridae (orthomyxoviruses such as influenza virus), the family Paramyxoviridae (paramyxoviruses such as the genus Morbillivirus), the family Rhabdoviridae (rhabdoviruses such as rabies virus), the family Filoviridae (filoviruses such as Ebola virus and Marburg virus) and the family Bunyaviridae (bunyaviruses such as hantavirus). The negative-strand RNA virus may preferably be a virus of the family Paramyxoviridae, preferably a paramyxovirus, and preferably a Sendai virus.

Using Sendai virus as an example, the constitution of a negative-strand RNA virus or a minus-strand RNA virus vector will be illustrated. The Sendai virus comprises a negative-strand RNA genome; and a nucleoprotein (N protein), a phosphoprotein (P protein), a matrix protein (M protein), a fusion protein (F protein), a hemagglutinin-neuraminidase protein (HN protein) and a large protein (L protein). Each of the N protein, P protein and L protein can form an RNP complex with the RNA genome, and the M protein, F protein and HN protein can constitute the outer shell of a virus particle. The genome is believed to be encapsulated in the outer shell. The Sendai virus can infect a cell through the F protein. By constructing the outer shell as to have the F protein and subjecting the genome to modification (such as deletion) so that it cannot produce the F protein, the Sendai virus that proliferates through the genome after infection lacks the F protein so that it cannot further propagate. The nucleoprotein (N protein), phosphoprotein (P protein) and large protein (L protein) are required for the autonomous replication of the virus genome or vector genome in the introduced cell. Examples of the phosphoprotein (P protein) include P proteins having amino acid sequences registered under GenBank Accession Nos. BAN84668.1, AAB06197.1, P04859.1, P14252.1, AAB06291.1, AAX07439.1, BAM62828.1, BAM62834.1, P04860.1, BAM62840.1, BAD74220.1, P14251.1, BAM62844.1, BAM62842.1, BAF73480.1, BAD74226.1, BAF73486.1, Q9DUE2.1, BAC79134.1, NP_056873.1 and ABB00297.1. In a preferred embodiment, examples of the phosphoprotein (P protein) include a P protein having an amino acid sequence set forth in GenBank Accession No. BAN84668.1 (SEQ ID NO: 1), and a P protein having a sequence corresponding to the above-described sequence. Examples of the large protein (L protein) include an L protein having an amino acid sequence set forth in GenBank Accession No. BAN84672.1 (SEQ ID NO: 5), and an L protein having a sequence corresponding to the above-described sequence. The P protein of Sendai virus can have an amino acid sequence corresponding to the amino acid sequence of any of the above-described P proteins. The amino acid number of the P protein referred to herein is the amino acid number in the amino acid sequence set forth in SEQ ID NO: 1. Even in P proteins having other amino acid sequences, those skilled in the art will be able to identify the amino acid corresponding to the amino acid of the amino acid number of interest by aligning the amino acid sequences with each other or by other means. The L protein of Sendai virus can have an amino acid sequence corresponding to the amino acid sequence of any of the above-described L proteins. The amino acid number of the L protein referred to herein is the amino acid number in the amino acid sequence set forth in SEQ ID NO: 5. Even in L proteins having other amino acid sequences, those skilled in the art will be able to identify the amino acid corresponding to the amino acid of the amino acid number of interest by aligning the amino acid sequences with each other or by other means.

As used herein, the "regulatory sequence" refers to a sequence that has the activity of driving a gene operably linked to it and transcribing RNA from the gene. The regulatory sequence is, for example, a promoter. Examples of the promoter include a class I promoter (which may be used for transcription of an rRNA precursor), class II promoters (composed of a core promoter and upstream promoter elements), and class III promoters (further classified broadly into type I, type II and type III).

In an embodiment, the negative-strand RNA virus vector has, on its genome, factors related to proliferation or infection to cells of the negative-strand RNA viruses disrupted (for example, deleted), and has a reduced proliferation or infection ability or no substantial proliferation ability in cells other than the packaging cell. As described above, such a vector is given an initial infectivity to cells by constructing it under conditions in which the packaging cells have been supplied with disrupted growth or infection-related factors. Thereby, the vector obtained from the packaging cell has the infectivity, but the vector that subsequently infects a cell other than the packaging cell cannot generate any more infectious particles, thereby leading to improvement in the safety of the vector.

As used herein, the term "exogenous" means "derived from an organism other than the host". The regulatory sequence may be typically exogenous. The genes to be expressed may be also exogenous. In a gene operably linked to a regulatory sequence, both the regulatory sequence and the gene may be exogenous. The term "endogenous" means "possessed by the host itself".

As used herein, the expression "corresponding to a particular amino acid of the protein A" means "present in a homolog or ortholog of the protein A at a position corresponding to that of the particular amino acid of the protein A when aligned".

### <Virus or virus vector of the present invention or RNA genome thereof>

According to the present invention, a negative-strand RNA virus or virus vector and an RNA genome thereof are provided. According to the present invention, the negative-strand RNA virus may be preferably a mononegavirus, more preferably a virus of the family Paramyxoviridae, even more preferably a virus of the genus Respirovirus of the family Paramyxoviridae, and most preferably Sendai virus. According to the present invention, virus vectors constructed from these viruses may be provided.

The virus or vector may comprise a negative-strand RNA genome (hereinafter sometimes simply referred to as "virus genome"), and a nucleoprotein (N protein), a phosphoprotein (P protein), a matrix protein (M protein), a fusion protein (F protein), a hemagglutinin-neuraminidase protein (HN protein) and a large protein (L protein). The virus or virus vector may comprise an RNP complex comprising an N protein, a P protein and L protein, and a virus genome or virus vector genome. The virus or virus vector may also have an outer shell of a virus particle containing an M protein, an F protein and an HN protein.

In an embodiment of the present invention, the virus or virus vector has at least a P protein. In this embodiment, the P protein is preferably comprised in a RNP complex. In this embodiment, the virus or virus vector preferably also has an outer shell of a virus particle containing an M protein, an F protein and an HN protein. The P protein may include its functional variants. Functional variants may be, for example, proteins having at least 90% sequence identity with the amino acid sequence of the P protein described in SEQ ID NO: 1 and having the function of a P protein. Functional variants include temperature-sensitive mutants of the P protein. Functional variants may also be fragments of the P protein. The fragments may be a C-terminal domain of the P protein, which includes the N-binding site (in the case of Sendai virus, amino acid region 479-568 of SEQ ID NO: 1) and the L-binding site (in the case of Sendai virus, amino acid region 411-445 of SEQ ID NO: 1), or a region that further includes the oligomerization site (in the case of Sendai virus, amino acid region 320-446 of SEQ ID NO: 1) in addition to the above region, or the C-terminal region (in the case of Sendai virus, amino acid region 320-568 of SEQ ID NO: 1) (see, for example, Blanchard L. et al., Virology (2004) 319, 201-211); a protein having at least 90% sequence identity with the amino acid sequence of the region and having the function of a P protein (e.g., tetramerization ability and/or function as a polymerase subunit). In an embodiment, the fragment is a C-terminal domain of the P protein, being a region including the N-binding site (amino acid region 479-568 of SEQ ID NO: 1 for Sendai virus) and the L-binding site (amino acid region 411-445 of SEQ ID NO: 1 for Sendai virus), or a region further including the oligomerization site (amino acid region 320-446 of SEQ ID NO: 1 for Sendai virus) in addition to the above region; or a protein having at least 90% sequence identity with the amino acid sequence of the region and having P protein function (e.g., tetramerization ability). In an embodiment, the fragment is a C-terminal domain of the P protein, being the C-terminal region (amino acid region 320-568 of SEQ ID NO: 1 for Sendai virus), or a protein having at least 90% sequence identity with the amino acid sequence of the region and having P protein function (e.g., function as a polymerase subunit).

Previous studies have revealed that the Sendai viruses or Sendai virus vectors constructed by replacing a P protein with a P protein mutant exhibit temperature sensitivity. Specifically, it has been shown that the P protein mutant (SEQ ID NO: 2), which contains the L511F mutation and all of the D433A/R434A/K437A mutations, is a temperature-sensitivity mutation and the virus or virus vector having the P protein mutant is eliminated from a cell by increasing the temperature (WO 2010/008054, 2017/082174).

Hereinafter, a temperature-sensitive mutant of a P protein and a temperature-sensitive mutant of an L protein will be described, and these mutants are described for a P protein and an L protein encoded by a virus genome. However, the P protein and L protein as component proteins of the virus or virus vector having the virus genome may or may not be temperature sensitive. That is, after infecting a target cell, the virus or virus vector proliferates in the cell using proteins translated from the virus genome, and the P protein and preferably the L protein of the virus or virus vector which proliferate in the cell will exhibit the desired effect if they are even temperature sensitive.

Therefore, in an embodiment, the P protein and L protein of the virus or virus vector having the virus genome may have a lower temperature-sensitivity than the temperature-sensitive mutant of the P protein and L protein encoded by the virus genome, and preferably they do not have the temperature sensitivity (for example, have the temperature sensitivity equivalent to that of the wild type). With such a constitution, the virus or virus vector is less likely to decrease in production amount at the culture temperature of a packaging cell and can be expected to increase in virus or virus vector production efficiency. In another embodiment, the P protein and L protein as protein components of the virus or virus vector having the virus genome may have a temperature-sensitive mutant of the P protein and preferably a temperature-sensitive mutant of the L protein, which will be described below. The presence or absence and strength of temperature sensitivity at a particular temperature can be determined based on the amount of the virus or virus vector after culturing it at a particular temperature.

A temperature-sensitive mutant of a P protein and a temperature-sensitive mutant of an L protein will be described. According to the present invention, the P protein encoded by a virus genome has a temperature-sensitivity mutation and can give temperature sensitivity to a virus or virus vector. In the present invention, the temperature sensitivity of the virus or virus vector significantly decreases in proliferation, significantly decreases in amount, or decreases in amount to or below the detection limit, in a particular temperature region, as compared to a virus or virus vector without any corresponding temperature-sensitivity mutation on its virus genome. The particular temperature region is not limited so long as it is a temperature at which cell proliferation is not inhibited, but it is, for example, about 35°C to about 39°C, and preferably 36°C to 38.5°C. In an embodiment of the present invention, the P protein has a substitution mutation(s) in an amino acid(s) of the P protein corresponding to one or more or all of D433, R434, and K437, and the substitution mutation(s) comprises a mutation to any of G, T, and S. That is, in an embodiment of the present invention, the P protein has the amino acid sequence set forth in SEQ ID NO: 4 {except for SEQ ID NOs: 1 and 2}. In this way, the P protein mutant can give temperature sensitivity to a Sendai virus or Sendai virus vector. In a preferred embodiment of the invention, the P protein mutant can exhibit the temperature sensitivity that is higher than a P protein mutant containing the D433A/R434A/K437A mutations.

In an embodiment of the present invention, the P protein has substitution mutations in the amino acids of the P protein corresponding to all of D433, R434, and K437, and each of the substitution mutations comprises a mutation to any of G, T, and S. In an embodiment of the present invention, the P protein has substitution mutations in the amino acids of the P protein corresponding to all of D433, R434, and K437, and each of the substitution mutations is a mutation to any of G, T, and S. In an embodiment of the present invention, the P protein has substitution mutations in the amino acids of the P protein corresponding to all of D433, R434, and K437, and each of the substitution mutations is a mutation to T. In an embodiment of the present invention, the P protein has substitution mutations in the amino acids of the P protein corresponding to all of D433, R434, and K437, and each of the substitution mutations is a mutation to S.

In an embodiment of the present invention, the P protein may have a substitution mutation in the amino acid sequence corresponding to L511. In an embodiment of the present invention, the amino acid sequence corresponding to L511 of the L protein is preferably mutated to F or Y.

For example, the P protein may be a P protein mutant having an amino acid sequence corresponding to the amino acid sequence set forth in SEQ ID NO: 3. In addition, for example, the P protein may be a P protein mutant having an amino acid sequence corresponding to the amino acid sequence set forth in SEQ ID NO: 4.

In an embodiment of the present invention, the P protein may have, in the amino acid corresponding to D433, a mutation to an amino acid selected from the group consisting of S, A, and T; it may have, in the amino acid corresponding to R434, a mutation to an amino acid selected from the group consisting of S, A, and T; it may have, in the amino acid corresponding to K437, a mutation to an amino acid selected from the group consisting of S, A, and T; and it may have, in the amino acid corresponding to L511, a mutation to F or Y. In another embodiment of the present invention, the P protein preferably:
(1S) has amino acid mutations corresponding to D433S, R434A, K437A and L511F;
(2S) has amino acid mutations corresponding to D433S, R434A, K437A and L511Y;
(3S) has amino acid mutations corresponding to D433A, R434S, K437A and L511F;
(4S) has amino acid mutations corresponding to D433A, R434S, K437A and L511Y;
(5S) has amino acid mutations corresponding to D433A, R434A, K437S and L511F;
(6S) has amino acid mutations corresponding to D433A, R434A, K437S and L511Y;
(7S) has amino acid mutations corresponding to D433S, R434S, K437A and L511F;
(8S) has amino acid mutations corresponding to D433S, R434S, K437A and L511Y;
(9S) has amino acid mutations corresponding to D433S, R434A, K437S and L511F;
(10S) has amino acid mutations corresponding to D433S, R434A, K437S and L511Y;
(11S) has amino acid mutations corresponding to D433A, R434S, K437S and L511F;
(12S) has amino acid mutations corresponding to D433A, R434S, K437S and L511Y;
(13S) has amino acid mutations corresponding to D433S, R434S, K437S and L511F;
(14S) has amino acid mutations corresponding to D433S, R434S, K437S and L511Y;
(1T) has amino acid mutations corresponding to D433T, R434A, K437A and L511F;
(2T) has amino acid mutations corresponding to D433T, R434A, K437A and L511Y;
(3T) has amino acid mutations corresponding to D433A, R434T, K437A and L511F;
(4T) has amino acid mutations corresponding to D433A, R434T, K437A and L511Y;
(5T) has amino acid mutations corresponding to D433A, R434A, K437T and L511F;
(6T) has amino acid mutations corresponding to D433A, R434A, K437T and L511Y;
(7T) has amino acid mutations corresponding to D433T, R434T, K437A and L511F;
(8T) has amino acid mutations corresponding to D433T, R434T, K437A and L511Y;
(9T) has amino acid mutations corresponding to D433T, R434A, K437T and L511F;
(10T) has amino acid mutations corresponding to D433T, R434A, K437T and L511Y;
(11T) has amino acid mutations corresponding to D433A, R434T, K437T and L511F;
(12T) has amino acid mutations corresponding to D433A, R434T, K437T and L511Y;
(13T) has amino acid mutations corresponding to D433T, R434T, K437T and L511F;
(14T) has amino acid mutations corresponding to D433T, R434T, K437T and L511Y;
(1ST) has amino acid mutations corresponding to D433S, R434T, K437S and L511F; or
(2ST) has amino acid mutations corresponding to D433S, R434T, K437S and L511Y.

In an embodiment of the present invention, the virus or virus vector has a gene encoding at least a P protein and an L protein. The P protein encoded by an RNA genome has any of the mutations as described above. The L protein encoded by an RAN genome may have a temperature-sensitivity mutation and may be able to give temperature sensitivity to the virus or virus vector. In the present invention, the temperature sensitivity of the virus or virus vector is defined as a significant decrease in its proliferation, a significant decrease in its amount, or its amount reaching below the detection limit, in a particular temperature region, as compared to a virus or virus vector without the corresponding temperature-sensitivity mutation. In an embodiment, the L protein preferably has a substitution mutation(s) in an amino acid(s) corresponding to one or more or all of N1197, L1361, L1558 and K1796. The amino acid mutation corresponding to N1197 is, for example, preferably a mutation to S. The amino acid mutation corresponding to L1361 is, for example, preferably a mutation to C. The amino acid mutation corresponding to NL1558 is, for example, preferably a mutation to I. The amino acid mutation corresponding to K1796 is, for example, preferably a mutation to E. Therefore, in an embodiment, the L protein preferably has any one or more or all (preferably all) of N1197, L1361C, L1558I, and K1796. In an embodiment, it is preferable that the L protein has any or preferably all of N1197S, L1558I, and K1796E, and more preferably further has L1361C. That is, in an embodiment of the present invention, the L protein has the amino acid sequence set forth in SEQ ID NO: 6 (except for SEQ ID NO: 5).

Therefore, in an embodiment of the present invention, it is preferable that: the above-described virus or virus vector has a gene encoding at least a P protein and an L protein; the P protein encoded by an RNA genome has any of the above-described mutations; and the L protein encoded by an RNA genome has any of the above-described mutations. That is, in an embodiment, it is preferable that: the P protein has an amino acid sequence set forth in in SEQ ID NO: 4 (except for SEQ ID NOs: 1 and 2); and the L protein has an amino acid sequence set forth in in SEQ ID NO: 6 (except for SEQ ID NO: 5). In an embodiment, it is preferable that: the P protein has substitution mutations in the amino acids of the P protein corresponding to all of D433, R434, and K437, and each of the substitution mutations comprises a mutation to any of G, T, and S; and the L protein has one or both (preferably both) of L1361C and L1558I. In an embodiment, it is preferable that: the P protein has substitution mutations in the amino acids of the P protein corresponding to all of D433, R434, and K437, and each of the substitution mutations comprises a mutation to any of G, T, and S; the P protein has a substitution mutation in L511 or an amino acid sequence corresponding to L511 and the substitution mutation is a mutation to F or Y; and the L protein has one or both (preferably both) of L1361C and L1558I. In an embodiment, it is preferable that: the P protein has substitution mutations in the amino acids of the P protein corresponding to all of D433, R434, and K437, and each of the substitution mutations is a mutation to any of G, T, and S; the P protein has a substitution mutation in L511 or an amino acid sequence corresponding to L511 and the substitution mutation is a mutation to F or Y; and the L protein has one or both (preferably both) of L1361C and L1558I. In an embodiment, it is preferable that the P protein has mutations described in any of (1S) to (14S) and (1T) to (14T) and (1ST) to (2ST) as described above and the L protein has one or both (preferably both) of L1361C and L1558I. The mutations in the P protein can be used in combination with mutations in other proteins. The mutations in the L protein can be also used in combination with mutations in other proteins.

In an embodiment of the present invention, the virus or virus vector has an M protein or a mutant thereof. The mutant of the M protein can have a substitution mutation(s) in an amino acid(s) of the M protein corresponding to one to three amino acids selected from the group consisting of G69, T116 and A183. The mutant of the M protein can have, for example, a mutation in the amino acid corresponding to G69, and the mutation can be, for example, a mutation corresponding to G69E. The mutant of the M protein can have, for example, a mutation in the amino acid corresponding to G116, and the mutation can be, for example, a mutation corresponding to T116A. The mutant of the M protein can have, for example, a mutation in the amino acid corresponding to A183, and the mutation can be, for example, a mutation corresponding to A183T or A183S, and preferably, a mutation corresponding to A183S. In an embodiment, the mutant of the M protein can have amino acid mutations corresponding to G69E, T116A, and A183T, or preferably, amino acid mutations corresponding to G69E, T116A, and A183S. The mutation in the M protein can reduce the cytotoxicity of SeV. The mutations in the M protein can be used in combination with mutations in other proteins.

In an embodiment of the present invention, the virus or virus vector has an HN protein or a mutant thereof. The mutant of the HN protein can have a substitution mutation(s) in an amino acid(s) of the HN protein corresponding to one to three amino acids selected from the group consisting of A262, G264 and K461. HN protein can have, for example, a mutation in the amino acid corresponding to A262, and the mutation can be, for example, a mutation corresponding to A262T. The HN protein can have, for example, a mutation in the amino acid corresponding to G264, and the mutation can have, for example, a mutation corresponding to G264R or G264K and preferably a mutation corresponding to G264K. The HN protein can have, for example, a mutation in the amino acid corresponding to K461, and the mutation can be, for example, a mutation corresponding to K461E. In an embodiment, the mutant of the HN protein can have amino acid mutations corresponding to A262T, G264R, and K461E, or preferably amino acid mutations corresponding to A262T, G264K and K461E. The mutation to the HN protein can reduce the cytotoxicity of SeV. The mutations in the HN protein can be used in combination with mutations in other proteins.

In one embodiment of the present invention, each of the P protein, L protein, M protein and HN protein can have the mutation(s) described above.

Each of the amino acid substitutions defined above may also be a substitution to a conservative amino acid with respect to the amino acid after such a mutation. For example, a mutation to an uncharged polar amino acid may be a mutation to another uncharged polar amino acid, and a mutation to a nonpolar amino acid may be a mutation to another nonpolar amino acid.

The term "conservative substitution" means a substitution of an amino acid within each group of:
the group of acidic amino acids (aspartic acid (D) and glutamic acid (E));
the group of basic amino acids (arginine (R), lysine (K) and histidine (H));
the group of uncharged polar amino acids (asparagine (N), glutamine (Q), serine (S), threonine (T) and tyrosine (Y)); and
the group of nonpolar amino acids (alanine (A), glycine (G), valine (V), leucine (L), isoleucine (I), proline (P), phenylalanine (F), methionine (M), tryptophan (W) and cysteine (C)), and
the term "nonconservative substitution" means an amino acid substitution that is not a conservative substitution.

In an embodiment, the virus or virus vector may further have a gene of interest (GOI). The loading position of the GOI on an RNA genome is before the N gene (hereinafter expressed as "+"), between the P gene and the M gene (hereinafter expressed as "PM"), between the M gene and the HN gene (hereinafter expressed as "MHN"), or between the HN gene and the L gene (hereinafter expressed as "HNL"), and the levels of gene expression are known to decrease in the order of these loading positions.

In an embodiment, the above-described virus or virus vector can comprise genomes containing genes encoding a nucleoprotein (N protein), a phosphoprotein (P protein), a matrix protein (M protein), a fusion protein (F protein), a hemagglutinin-neuraminidase protein (HN protein) and a large protein (L protein). In an embodiment, it does not contain a gene encoding a functional fusion protein (F protein) (and preferably having a deletion of such a gene), and can contain genomes containing encoding genes encoding a nucleoprotein (N protein), a phosphoprotein (P protein), a matrix protein (M protein), a hemagglutinin-neuraminidase protein (HN protein) and a large protein (L protein). In all embodiments of the present invention, each of the gene encoding a P protein on a genome and optionally the gene encoding an L protein preferably has the temperature-sensitivity mutation as described above. In this way, the virus or virus vector that has proliferated in a cell after infection can exhibit the of temperature sensitivity. In all embodiments of the present invention, on the virus or virus vector particle, the P protein and L protein may or not have temperature-sensitivity mutation. The virus or virus vector may be of pseudo-type. The virus or virus vector of pseudo-type refers to one having an envelope protein thereof changed, and may have the envelope protein changed to one derived from another virus. This makes it possible to change the cell tropism of the virus. In a preferred embodiment, the genome of virus or virus vector has a P protein and/or an L protein having the above-described temperature-sensitivity mutation, and the virus particle itself of the virus or virus vector may contain a wild-type P protein and /or a wild-type L protein or may also even contain no temperature-sensitivity P protein and no temperature-sensitivity L protein.

In an embodiment, the above-described virus or virus vector has an RNP complex containing a genome and a nucleoprotein (N protein), a phosphoprotein (P protein) and a large protein (L protein); and a virus particle containing a matrix protein (M protein), a fusion protein (F protein) and a hemagglutinin-neuraminidase protein (HN protein); the virus particle contains the complex.

In an embodiment, the above-described virus or virus vector can contain a gene of interest operably linked to a regulatory sequence. The above-described virus or virus vector can express, in an infected cell, the gene of interest operably linked to a regulatory sequence. The gene of interest can be transcribed into RNA, the RNA exhibiting physiological activities, or it can be translated via RNA into a protein, the protein exhibiting physiological activities. The term "regulatory sequence" refers to a sequence that has the activity of driving a gene operably linked thereto and transcribing RNA from the gene. The gene of interest may be exogenous to a virus or virus vector. The gene of interest may be also exogenous to a cell to be infected. As used herein, the term "exogenous" means that the particular species does not inherently have it. The regulatory sequence is, for example, a promoter. Examples of the promoter include a Class I promoter (capable of being used for transcription of an rRNA precursor), a Class II promoter (composed of a core promoter and a promoter element upstream thereof, and capable of being used for transcription of mRNA), and a Class III promoter (further broadly classified into types I, II and III).

The above-described virus or virus vector can infect an animal cell (particularly a mammalian cell, preferably a human cell). The above-described virus or virus vector can preferably amplify its genome in an animal cell (particularly a mammalian cell, preferably a human cell). The above-described virus or virus vector can preferably exhibit temperature sensitivity at a temperature of 37°C or more, 37.5°C or more, 38°C or more, or 38.5°C or more. This temperature sensitivity allows the virus or virus vector to be eliminated from the cell.

The above-described virus or virus vector can be produced by a conventional method for producing a virus or virus vector. When producing it, it is possible to use, as a gene encoding a P protein, a gene encoding any of the P protein mutants as described above. In addition, when producing it, it is possible to use, as a gene encoding an L protein, a gene encoding any of the L protein mutants as described above. The RNA genome of the above-described virus or virus vector can be obtained by transcription from a nucleic acid encoding the RNA genome of the virus or virus vector (preferably DNA, more preferably an expression plasmid containing the nucleic acid). It is preferable that a nucleic acid having a region encoding the RNA genome of the above-described virus or virus vector further has a regulatory sequence and the region is operably linked to the regulatory region. The nucleic acid having a region encoding the RNA genome of the above-described virus or virus vector is preferably loaded on an expression vector for the nucleic acid. Examples of the expression vector include, but not particularly limited to; a plasmid vector, a virus vector, a cosmid vector, and an artificial chromosome. The expression vector may further have, in addition to an expression cassette of an RNA genome operably linked to a regulatory sequence, an origin of self-replication (Ori) and a positive selection marker gene (such as a drug resistance gene or a gene encoding a fluorescent protein). RNA genomes of viruses or virus vectors are known to be produced in packaging cells that supply protein components of the viruses or the other components. The packaging cell will supply components contained in the virus or virus vector particle to be produced. The virus or virus vector particle is preferably produced in large quantities in a packaging cell. From this viewpoint, the protein supplied to the packaging cell preferably does not have the temperature sensitivity as described in the present specification, and is preferably a wild-type protein. The titer of the virus thus produced can be measured based on a conventional method. The titer may be 1 × 10⁵ CIU/mL or more, 2 × 10⁵ CIU/mL or more, 3 × 10⁵ CIU/mL or more, 4 × 10⁵ CIU/mL or more, 5 × 10⁵ CIU/mL or more, 6 × 10⁵ CIU/mL or more, 7 × 10⁵ CIU/mL or more, 8 × 10⁵ CIU/mL or more, 9 × 10⁵ CIU/mL or more, 1 × 10⁶ CIU/mL or more, 2 × 10⁶ CIU/mL or more, 3 × 10⁶ CIU/mL or more, 4 × 10⁶ CIU/mL or more, 5 × 10⁶ CIU/mL or more, 6 × 10⁶ CIU/mL or more, 7 × 10⁶ CIU/mL or more, 8 × 10⁶ CIU/mL or more, 9 × 10⁶ CIU/mL or more, 1 × 10⁷ CIU/mL or more, 2 × 10⁷ CIU/mL or more, 3 × 10⁷ CIU/mL or more, 4 × 10⁷ CIU/mL or more, or 5 × 10⁷ CIU/mL or more.

### <Method for culturing cell infected with the above virus or virus vector>

According to the present invention, a method for infecting a cell with a virus or virus vector, and a method for culturing the cell infected with the virus or virus vector are provided.

According to the present invention, the method for infecting a cell with a virus or virus vector comprises contacting the cell with the virus or virus vector of the present invention as described above in an environment suitable for infection. By loading the virus or virus vector with a selection marker gene (for example, a visualization marker gene such as a fluorescent protein, and a drug resistance gene), the cell infected with the virus or virus vector can be selected using the expression of the selection marker gene as an indicator.

According to the present invention, a method for culturing a cell infected with the virus or virus vector of the present invention is provided. The method for culturing a cell infected with the virus or virus vector of the present invention comprises culturing the cell infected with the virus or virus vector of the present invention in an environment suitable for maintaining or proliferating the cell. The cell can be infected with the virus or virus vector of the invention as appropriate using a conventional method by those skilled in the art. According to the present invention, a cell infected with the virus or virus vector of the invention can be also provided.

In an embodiment, the method for culturing a cell infected with the virus or virus vector comprises culturing the cell under the first temperature conditions. In an embodiment, the method for culturing a cell infected with the virus or virus vector can comprise culturing the cell under the first temperature conditions and then culturing the cell under the second temperature conditions.

The first temperature conditions are conditions suitable for maintaining or proliferating the virus or virus vector that has infected the cell.

On the other hand, the second temperature conditions are conditions suitable for allowing the virus or virus vector to decrease in amount or disappear.

The first temperature is a temperature lower than the second temperature.

When maintaining the virus or virus vector of the present invention in a cell, for example, the cell infected with the virus or virus vector of the present invention can be cultured under the first temperature conditions. When allowing the virus or virus vector of the present invention to decrease or disappear, for example, the cell infected with the virus or virus vector of the present invention can be cultured under the second temperature conditions. The virus or virus vector of the present invention can be maintained or proliferated in the infected cell, but the virus or virus vector can be inhibited from being maintained or proliferated and can be eliminated from the infected cell, by increasing the culture temperature. The first and second temperatures can be determined as appropriate by those skilled in the art.

The method for culturing a cell infected with the virus or virus vector can comprise culturing the cell under the conditions suitable for maintaining or proliferating the virus or virus vector. The conditions suitable for maintaining or proliferating the virus or virus vector preferably comprise temperature conditions under which the virus or virus vector is not affected by the temperature sensitivity due to mutations in a P protein or an L protein. For example, when it is necessary to allow the virus or virus vector of the present invention to be maintained or proliferated in a cell (for example, when the gene of interest loaded on the virus or virus vector is allowed to be highly expressed), the infected cell can be cultured under the conditions suitable for maintaining or proliferating the virus or virus vector. The temperature conditions under which the virus or virus vector is not affected by the temperature sensitivity due to mutations in a P protein or an L protein can be determined as appropriate by those skilled in the art. For example, if the infected cell is not affected by the temperature sensitivity at 37°C, it can be cultured at 37°C. For example, if the infected cell is affected by the temperature sensitivity at 37°C, it can be cultured at a lower temperature, for example, under the temperature conditions of 35°C or more and less than 37°C. However, the conditions suitable for maintaining or proliferating the virus or virus vector of the present invention are those which do not have a significant adverse effect on maintaining or proliferating the cell.

The method for culturing a cell infected with a virus or virus vector comprises culturing the cell under the conditions suitable for allowing the virus or virus vector to decrease in amount. In an embodiment, the method for culturing a cell infected with a virus or virus vector can comprise culturing the cell under the conditions suitable for maintaining or proliferating the virus or virus vector, and then culturing the cell under the conditions suitable for allowing the vector or vector virus to decrease in amount. The conditions suitable for allowing the vector or vector virus to decrease in amount preferably comprises the temperature conditions under which the virus or virus vector is affected by the temperature sensitivity due to mutations in a P protein or an L protein. For example, when it is necessary to allow the virus or virus vector of the present invention to decrease in a cell (for example, when the gene of interest loaded on the virus or virus vector is allowed to express and then eliminated), the infected cell can be cultured under the conditions suitable for allowing the virus or virus vector to decrease. The temperature conditions under which the virus or virus vector is affected by the temperature sensitivity due to mutations in a P protein or an L protein can be determined as appropriate by those skilled in the art. For example, if the infected cell is not affected by the temperature sensitivity at 37°C, it can be cultured at a temperature above 37°C (for example, a temperature that is above 37°C and 39°C or less such as a temperature of 37.5°C to 38.5°C). For example, if the infected cell is affected by the temperature sensitivity at 37°C, it can be cultured at 37°C or more. In such a manner, the infected cell is cultured at a temperature (the first temperature) at which it is not affected or not easily affected, and when the virus or virus vector is to be eliminated from the infected cell, the culture temperature can be increased to the second temperature. However, the conditions suitable for allowing the virus or virus vector of the present invention to decrease are those that do not have a significant adverse effect (particularly, irreparable injury or damage, or the like) on maintaining or proliferating the cell. In this way, the virus or virus vector can be eliminated from the cell infected with the virus or virus vector. Therefore, in an embodiment, a method for obtaining a cell, in which a virus or virus vector is absent, from a cell infected with the virus or virus vector is provided. The absence of a virus or virus vector in a cell can be tested, for example, by detecting no genome of the virus or virus vector in the cell. Detection of a genome can be determined using a well-known technique, such as polymerase chain reaction (PCR), as appropriate by those skilled in the art. According to the method for culturing a cell as described above, transient gene transfer and gene expression can be achieved.

According to the present invention, the cell containing or infected with the virus or virus vector may be cultured at the second temperature from the beginning. That is, it takes time for the virus or virus vector to be eliminated from the infected cell. Therefore, the gene or the like loaded by the virus or virus vector can be expressed by the time that it is eliminated, resulting in achievement of the desired purpose. Therefore, the cell may not be cultured at the first temperature, but it may be cultured only at the second temperature.

Each of the first temperature and the second temperature need only be within a certain temperature range, but it may be preferably a certain temperature (± error range). The certain temperature range is a temperature range suitable for maintaining or proliferating the virus or virus vector for the first temperature, and is a temperature range (a lower temperature region) suitable for eliminating (decreasing) the virus or virus vector for the second temperature. For the purpose of further maintaining or culturing of the cell after eliminating the virus or virus vector, neither temperature range is a temperature that significantly harms cell survival nor significantly injures a cell. Preferably, both of the temperature ranges are temperatures that do not substantially affect cell survival.

According to the present invention, provided is a method for inducing differentiation of a cell, comprising;
infecting the cell with the virus or virus vector of the present invention;
then culturing the infected cell to induce the differentiation of the cell;
after inducing the differentiation, increasing the culture temperature to eliminate the virus or virus vector of the present invention from the differentiation-induced cell;
wherein the virus or virus vector has a differentiation-inducing factor expressibly loaded thereon. The term "inducing differentiation" means differentiating a cell into another stable cell state. Therefore, "a method for inducing differentiation of a cell" is used synonymously with "a method for producing a differentiation-induced cell. Various known methods can be used as a method for differentiating a cell into another stable cell state. The virus or virus vector of the present invention can have thereon a differentiation-inducing factor that promotes inducing differentiation. A cell can be induced to differentiate into a somatic cell such as a neuron, a cardiomyocyte, a chondrocyte, a bone cell, a hematopoietic cell (such as a leukocyte), a hepatocyte or a pancreatic beta cell. A cell can be induced to differentiate into such a somatic cell by a method known to those skilled in the art.

According to the present invention, provided is a method for reprogramming (nuclear reprogramming) of a cell, comprising:
infecting the cell with the virus or virus vector of the present invention;
then culturing the infected cell to reprogram the cell; and
after inducing the differentiation, increasing the culture temperature to eliminate the virus or virus vector of the present invention from the reprogrammed cell;
wherein the vector or virus vector has a reprogramming factor expressibly loaded thereon. Examples of the reprogramming factor include, but not particularly limited to, Yamanaka factors (OCT4, SOX2, KLF4, and optionally c-MYC), Thomson factors (OCT4, SOX2, NANOG, and LIN-28), and other reprogramming factors. The reprogramming factor may be, for example, KLF4, OCT4, SOX2 and MYCL.

The present invention can be used, without being limited to the above, for various applications in which it is desired to transiently express as a protein or RNA followed by elimination of a vector. The present invention could be also used for expressing, in a cell, a protein component and if necessary an RNA component for a genome editing system (for example, a zinc finger nuclease, TALEN, and a CRISPR/Cas system (such as a CRISPR/Cas9 system)), and, an RNA-protein complex (an RNP complex) if necessary, and editing a nucleic acid (such as a DNA) in a genome or the like of the cell and eliminating the vector after editing.

### <Elimination of virus or virus vector from the infected cell using degron>

In the present invention, a virus or virus vector that has infected a cell can also be eliminated from the infected cell using a degron. The degron may be used in combination with elimination of a virus or virus vector utilizing a temperature-sensitive mutant and a temperature change described above. Various degrons may be used, for example, degrons derived from a FK506 binding protein (FKBP12) and degrons derived from dihydrofolate reductase (DHFR) (see US 2009/0215169 A). The destabilizing domains (DD) of these proteins are degradation-inducible and can be used to fuse them with other proteins (proteins of interest) to induce degradation of the proteins of interest. For example, a DD tag and a degradation-inducing variant thereof can be added to a protein at its N- or C-terminal.

In the present invention, the FKBP12-derived DD may have an amino acid mutation corresponding to any amino acid mutation selected from the group consisting of F15S, V24A, H25R, E60G, L106P, D100G, M66T, R71G, D100N, E102G, and K105I. The FKBP12-derived DD preferably has a mutation in the amino acid corresponding to F36, particularly F36V. The FKBP12-derived DD also preferably has L106P. The FKBP12-derived DD also preferably has F36V and L106P. In the present invention, it preferably has one or more mutations preferably selected from the group consisting of 4I, 18K, 36L, 50I, 57K, 59F, 63A, 68L, 90V, 98I, and 105R, and it more preferably has all of these mutations.

According to the present invention, a gene encoding an N protein, a P protein, an M protein, an F protein, an HN protein, and an L protein fused with the above-described DD, and a gene encoding any of these is provided. According to the present invention, a cell or a Sendai virus or Sendai virus vector or a Sendai virus genome, containing one or more proteins selected form an N protein, a P protein, an M protein, an F protein, an HN protein and an L protein fused with the above-described DD and/or one or more genes encoding these proteins is provided. Each of these fusion proteins may contain, instead of each protein described above, a mutant thereof. The mutants are as described in the present specification.

According to the present invention, a P protein fused with the above-described DD and a gene encoding the P protein are provided. According to the present invention, a cell or a Sendai virus or Sendai virus vector or a Sendai virus genome, containing a P protein fused with the above-described DD and/or a gene encoding the P protein are provided. Here, a mutant of the P protein described in the present specification may be used instead of the P protein.

If the P protein fused with the above-described DD is essential for proliferating a negative-strand RNA virus or vector in a cell, the negative-strand RNA virus or vector can be eliminated from the cell in the presence of a degradation-promoting molecule such as dTAG-13 or a derivative thereof. If a negative-strand RNA virus or vector is to be maintained in a cell, the cell can be cultured in the presence of a stabilizing molecule such as Shield-1 or a derivative thereof. Thus, in the present invention, elimination (and preferably maintenance) of the virus or virus vector can be regulated by using a degradation-promoting molecule and preferably by further using a stabilizing molecule. dTAG-13 is a PROTAC (abbreviation for Proteolysis Targeting Chimera) having a binding site for the above-described DD or mutant thereof and a binding site for a ubiquitin E3 ligase, and induces degradation of a protein fused with the DD or mutant thereof.

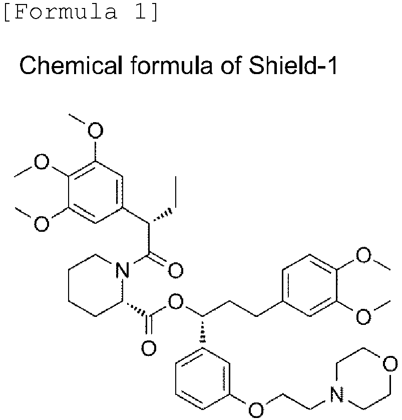

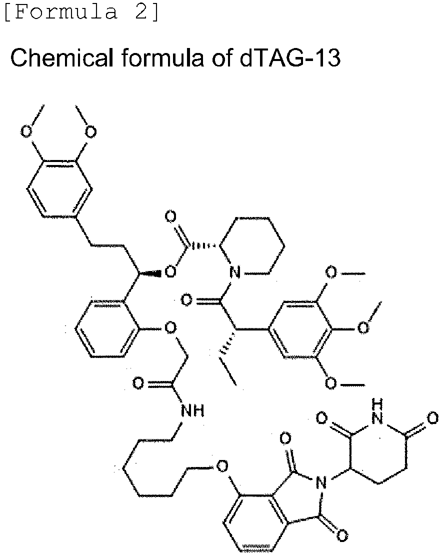

As a degron, a bromotag can also be used (Bond et al., J. Med. Chem., 2021, 64, 20, 15477-15502). Examples of the bromotag include Brd4^{BD2 (L387A)}, which is a peptide having the amino acid sequence of SEQ ID NO: 29, and a portion of the Brd4 protein having a corresponding amino acid sequence. The bromotag has protein degradation-inducing activity and can be used to induce degradation of another protein (target protein) by fusing it with the target protein. The bromotag may have at least 80%, 85%, 90%, 95%, 96%, 97%, 98%, 99%, or 100% sequence identity with the amino acid sequence of SEQ ID NO: 29 and the ability to induce degradation of the target protein fused with the tag in the presence of a bromotag ligand. The bromotag can be added to the N-terminus or C-terminus of a protein. In this manner, a fusion protein of the target protein and the bromotag can be obtained.

The present invention provides N protein, P protein, M protein, F protein, HN protein, and L protein fused with the above bromotag, as well as genes encoding any of these. The present invention further provides a cell, Sendai virus, Sendai virus vector, or Sendai virus genome, comprising one or more of the above bromotag-fused proteins selected from the group consisting of N protein, P protein, M protein, F protein, HN protein, and L protein, and/or one or more genes encoding them. These fusion proteins may include variants of the respective proteins described above. The variants are as described herein.

In particular, the present invention provides P protein fused with the above bromotag and a gene encoding the P protein. The present invention further provides a cell, Sendai virus, Sendai virus vector, or Sendai virus genome, comprising the above bromotag-fused P protein and/or a gene encoding the P protein. In place of the P protein, variants of the P protein described herein may be used. In one embodiment, the bromotag is fused to the C-terminus of the P protein. In one embodiment, the bromotag is fused to a temperature-sensitive modified P protein. In one embodiment, the temperature-sensitive modified P protein may be any described above. In one embodiment, the bromotag is fused to the C-terminus of the temperature-sensitive modified P protein. Examples of temperature-sensitive modified P proteins include P2m3SY, P2m2S434TY, and P2m3T. Bromotag-fused temperature-sensitive mutants may have an amino acid sequence of any one of SEQ ID NOs: 30-32. In one embodiment, the bromotag is fused to a temperature-sensitive modified L protein. In one embodiment, the bromotag is fused to the C-terminus of the temperature-sensitive modified L protein. In one embodiment, the temperature-sensitive modified L protein may be any described above.

When P protein fused with the above bromotag is essential for intracellular replication of a negative-strand RNA virus or vector, the virus or vector can be removed from the cell in the presence of a degradation-promoting molecule such as a bromotag ligand (e.g., AGB1) or a derivative thereof. Thus, in the present invention, the removal (and preferably maintenance) of the virus or viral vector in cells can be controlled using a degradation-promoting molecule. The bromotag ligand (e.g., AGB1) is a PROTAC (Proteolysis Targeting Chimera) having a site that binds to the above bromotag or its variant and a site that binds to ubiquitin E3 ligase, and induces degradation of the bromotag or variant fusion protein. AGB1 is (S)-13-((2S,4R)-4-hydroxy-2-(((4-(4-methylthiazol-5-yl)benzyl)carbamoyl)pyrrolidin-1-carbonyl))-14,14-dimethyl-11-oxo-3,6,9-trioxa-12-azapentadecyl (R)-2-(((S)-4-(4-chlorophenyl)-2,3,9-trimethyl-6H-thieno[3,2-f][1,2,4]triazolo[4,3-a][1,4]diazepin-6-yl)butanoate.

### <Packaging cell and its production method>

In the present invention, an existing packaging cell can be used to construct a negative-strand virus or vector such as SeV. However, in the present invention, a packaging cell can be newly constructed, and the obtained packaging cell may be used to produce a virus or vector.

In an embodiment, a packaging cell is a cell line, and preferably a stably established cell line. The packaging cell may be any type of cell that can be infected with a negative-strand RNA virus or vector, including, but not particularly limited to, an LLC-MK2 cell and a Vero cell. The packaging cell typically expresses one or more of the components of the virus such as an F protein. The packaging cell may additionally have a PKR inhibitory factor (such as VAI) loaded thereon.

The packaging cell has a gene encoding one or more of the components of the virus such as an F protein, and preferably exhibits expression at a certain level or more for the gene. The gene is operably linked to a regulatory sequence and its expression is driven by the regulatory sequence. The level of expression is determined by various factors such as a regulatory sequence, the location on a genome at which the gene is integrated, or the copy number of the gene inserted in the genome. In order to ensure a high expression level, marker genes (for example, a drug resistance gene and a visualization marker gene (such as a fluorescent protein)) linked downstream of the same promoter can be utilized during gene transfer. That is, in order to obtain a high-expressing cell line, a regulatory sequence first has a marker gene operably linked through a first site-specific recombinant sequence, and a gene encoding one or more components of a virus such as F protein, or the like is further linked thereto through a second site-specific recombinant sequence. In this constitution, the marker gene will be present between the first site-specific recombinant sequence and the second site-specific recombinant sequence (referred to as an intervening sequence). The first site-specific recombinant sequence and the second site-specific recombinant sequence can be recombined in the presence of a recombinase to eliminate the sequence (intervening sequence) therebetween. After eliminating the intervening sequence by the recombinase, a gene encoding one or more components of the virus such as an F protein is designed to be driven by the above-described regulatory sequence. In addition, the maker gene is present in the intervening sequence, and a cell strongly expressing the marker gene can be selected based on the expression intensity of the marker gene. If the marker gene is a drug resistance gene, a cell that expresses the drug resistance gene at a certain level or more is selected by selecting the cell with a certain concentration of the drug. If the marker gene is a visualization marker gene, a cell that expresses the visualization marker gene at a certain level or more is selected by measuring the expression intensity of the visualization marker gene (for example, if the visualization marker gene is GFP, by measuring the fluorescence intensity thereof). In such a manner, a cell having a high expression intensity of the marker gene is selected. In the selected cell, the above-described gene is suggested to be highly expressed due to various factors such as the location on a genome at which the gene is integrated or the copy number of the gene inserted in the genome. The selected cell is cloned, and the resulting clone is allowed to be acted on by a site-specific recombinase that recognizes the above-described site-specific recombinant sequence and induces recombination. Then, the intervening sequence is eliminated, and a gene encoding one or more components of the virus such as a F protein is operably linked to the above-described regulatory sequence. In such a manner, the packaging cell can be obtained. For example, the site-specific recombinase can be introduced into a cell by a negative-strand RNA virus vector that can be eliminated, such as a Sendai virus vector (such as a temperature-sensitive SeV). After introducing the site-specific recombinase, the vector can be eliminated to the detection limit or less, and the cell can be thereby preferably utilized as a packaging cell. After eliminating the intervening sequence, one site-specific recombinant sequence may remain between the above-described regulatory sequence and the introduced gene.

The site-specific recombinase and a recognition sequence thereof that can be used include, but not particularly limited to, a site-specific recombination reaction system such as a system of a Cre recombinase (hereinafter sometimes simply referred to as "Cre") as a site-specific recombinase and loxP as a recognition sequence thereof; a system of a Flp recombinase (hereinafter sometimes simply referred to as "Flp") as a site-specific recombinase and FRT as a recognition sequence; a system of a Dre recombinase and rox as a recognition sequence thereof (Anastassiadis, K. et al., Dis. Model. Mech. (2009) 2: 508 - 515); or a system of a ϕC31 recombinase and attP/attB as a recognition sequence thereof (Belteki G et al., Nat. Biotechnol. (2003) 21: 321 - 324). A SCre-SloxP system and a VCre-VloxP system (E. Suzuki and M. Nakayama, Nucleic Acid Res. (2011) 39 (8): e49), which are modified Cre-loxP systems, can also be used. A mutated FRT sequence such as FRT sequences described in International Publication No. WO 2001/023545 (for example, FRT (f2161) and FRT (f2262)), and a mutated loxP sequence such as a loxP sequence described in Japanese Patent Laid-Open No. H11-196880 may be used, and recognition sequences of such site-specific recombinases can be freely selected and used by those skilled in the art.

According to the present invention, a genome of a negative-strand RNA virus or vector containing a nucleic acid encoding any of the above-described proteins is provided. According to the present invention, a nucleic acid encoding the genome and a vector containing the nucleic acid are provided. The vector may be a cloning vector and an expression vector for the genome. In a preferred embodiment, the expression vector can transcribe the genome in a packaging cell.

### Examples

### Example 1: Preparation of negative-strand RNA virus vector

A negative-strand RNA virus vector was expressed in a virus packaging cell. In particular, the temperature sensitivity of mutagenesis into a P protein during expression, was evaluated.

A Sendai virus (SeV) was used as the negative-strand RNA virus vector. The Sendai virus was recovered from a culture supernatant three days after introducing a plasmid mixture into an F gene-expressing cell. The plasmid mixture contained pSeV-EmGFP on which a Sendai virus genome operably linked to a T7 promoter was loaded. The Sendai virus genome was loaded with a gene encoding EmGFP so that the proliferation of the genome was able to be detected by fluorescence. The Sendai virus genome was one deleted in the F gene.

### (1) The F gene-expressing cell was constructed as follows.

An F gene (having Kozak sequence added and being optimized to a human codon) of the SeV was loaded on pCAGGS-neo to construct pCAGGS-F-neo. A Vero cell or LLC-MK2 cell using ViaFect (Promega) was transfected with the obtained plasmid and selected using 1 to 2 mg/mL G418 disulfate solution (NACALAI TESQUE, INC.) to obtain an F gene-expressing cell. Each of the obtained cells is represented as Vero-F or LLC-MK2-F.

### (2) An F gene-deleted SeV was constructed as follows.

pSeV/dF in which the F gene-deleted SeV genome is to be transcribed with a T7 promoter was constructed on the basis of the gene sequence information of the SeV-Z strain of ACCESSION: AB855655. The following amino acid mutations were added to pSeV/dF in order to minimize the cytotoxicity of the SeV-Z strain: M protein: 69E, 116A and 183S; HN protein: 262T, 264R and 461E; and L protein: 1197S and 1796E (see, for example, International Publication No. WO 2003/025570). EmGFP, a mutant of GFP, was loaded on the genome as a gene of interest (GOI) to evaluate the temperature sensitivity and elimination of SeV. The gene expression level decreases depending on the GOI-loaded position in the following order: before an N gene (hereinafter designated as "+"), between a P gene and an M gene (hereinafter designated as "PM"), and between the M gene and an HN gene (hereinafter designated as "MHN") and between the HN gene and an L gene (hereinafter designated as "HNL"). The loading position of the EmGFP gene used was mainly the + position, that is, before the N gene of SeV. In the present Example, a mutation(s) by amino acid substitution was/were introduced into one or more of D433, R434, K437, and L511 of the P protein having the amino acid sequence set forth in SEQ ID NO: 1 on the SeV genome. In the packaging cell, both a P protein having temperature sensitivity and a P protein having no temperature sensitivity (such as a wild-type P protein) can be used, but the P protein having no temperature sensitivity (such as a wild-type P protein) was higher in production efficiency of the virus. Therefore, in the present Example, the gene encoding the P protein having a temperature sensitivity mutation was loaded on the genome, while the packaging cell was allowed to express the wild-type P protein and the wild-type P protein was introduced into a virus particle.

### (3) A plasmid for SeV reconstruction was prepared as follows.

With reference to WO 2005/071092, the addition of a Kozak sequence and the optimization to a human codon were performed, and pCAGGS-NPco, pCAGGS-P4C(-)co, pEF1-Lco and pCAGGS-F5Rco were constructed. 430P, 849I, and 880Y mutations were introduced into T7 with reference to P2001-54387A, and 644Y and 667Y mutations were introduced into T7 with reference to P2003-61683A, and pCAGGS-T7mco having the resulting T7m sequence loaded thereon was constructed. P4C(-) is a mutation in which the expression of a C protein from the P locus had disappeared.

The types of mutations in a P protein and the names of the mutant strains are as follows.
P2 : 433A, 434A, 437A, 511F (SEQ ID NO: 2)
P2Y : 433A, 434A, 437A, 511Y (SEQ ID NO: 8)
P2m3SY : 433S, 434S, 437S, 511Y (SEQ ID NO: 9)
P2m3TY : 433T, 434T, 437T, 511Y (SEQ ID NO: 10)
P2m3GY : 433G, 434G, 437G, 511Y (SEQ ID NO: 11)
P2m3SF : 433S, 434S, 437S, 511F (SEQ ID NO: 12)
P2m433S : 433S, 434A, 437A, 511F (SEQ ID NO: 13)
P2m433SY : 433S, 434A, 437A, 511Y (SEQ ID NO: 14)
P2m434SY : 433A, 434S, 437A, 511Y (SEQ ID NO: 15)
P2m437SY : 433A, 434A, 437S, 511Y (SEQ ID NO: 16)

The ratio of the plasmid for SeV reconstruction and a transfection reagent was in accordance with WO 2005/071092. Specifically, the following weights of the plasmid and the transfection reagent (TransIT-LT1 Reagent or ViaFect) were mixed to obtain a plasmid mix.

NP, P4C(-), F5R, T7: 0.5 µg each
L: 2 µg
pSeV: 5 µg
Total plasmids: 9 µg
TransIT-LT1 Reagent or ViaFect: 15 µL

Hereinafter, unless otherwise specified, the volume of the transfection reagent and the plasmid were mixed to provide a ratio of the volume of the transfection reagent and the weight of the plasmid of 9:15.

The plasmid and the transfection reagent were mixed into 225 µL of Opti-MEM, and the mixture was transfected into an F gene-expressing cell in a 12-well plate, which was being cultured in 500 µL of a medium (10% FBS/E-MEM), and was cultured at 37°C. The cells were cultured at 32°C from the day after transfection. The culture medium was exchanged daily with a serum-free medium (ITS-X/NEAA/E-MEM) supplemented with 2.5 µg/mL trypsin. The culture supernatant on the third day after transfection was recovered, the F gene-expressing cell that had seeded in a culture flask was infected therewith, and the SeV was amplified. The culture supernatants on the third day to the fifth day was recovered from the flask, diluted 10 times to 100,000 times, and a Vero cell that had been seeded in a 96-well plate was infected therewith. The number of GFP-positive cells was counted 3 days after infection using a fluorescence microscope system ECLIPSE Ti2-E (NIKON CORPORATION), and the infectious titer was calculated.

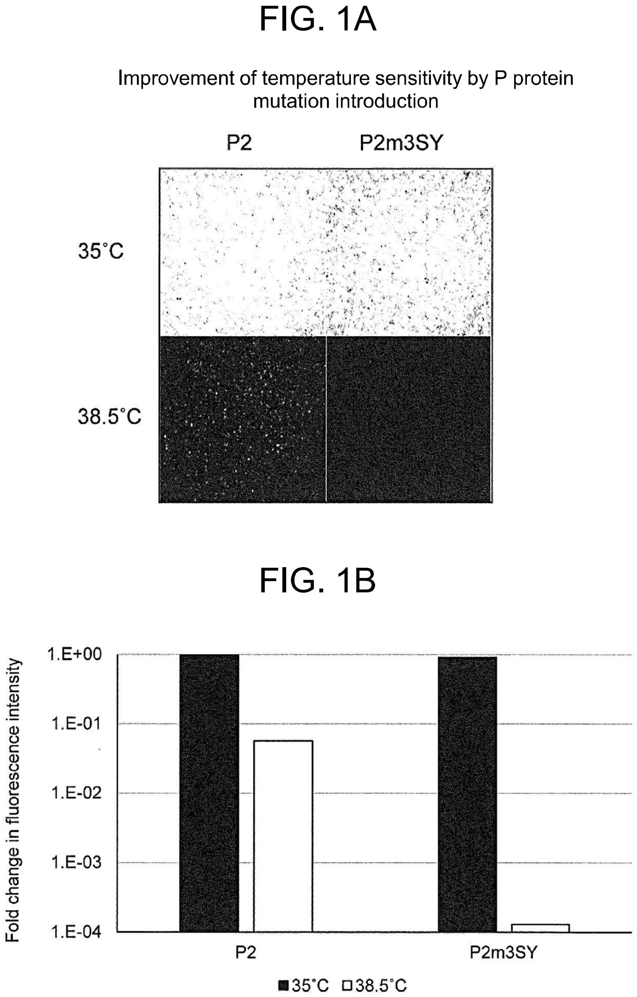

The Vero cell was infected with the resulting SEV mutant (MOI = 20). The infected cell was cultured under the temperature conditions of 35°C or 38.5°C for three days. Since the SeV vector expresses EmGFP in a cell and the infected cell emits fluorescence depending on the amount of SeV in the cell, the amount of SeV in the infected cell was estimated by measuring the fluorescence from the cell. The results were as shown in Figures 1A and 1B. As shown in Figure 1A, the P2m3SY mutant exhibited a strong fluorescence at 35°C, and the fluorescence strongly decreased at 38.5°C. Figure 1B shows the numerical value of the amount of fluorescence. The P2m3SY mutant decreased in amount of fluorescence to about 0.013% at 38.5°C compared to at 35°C. The P2 mutant decreased in amount of fluorescence to about 5.7% compared to at 35°C. This indicates that the P2m3SY mutant has a temperature sensitivity higher by 400 times or more than that of the P2 mutant.

### (4) Introduction of further temperature-sensitivity mutation

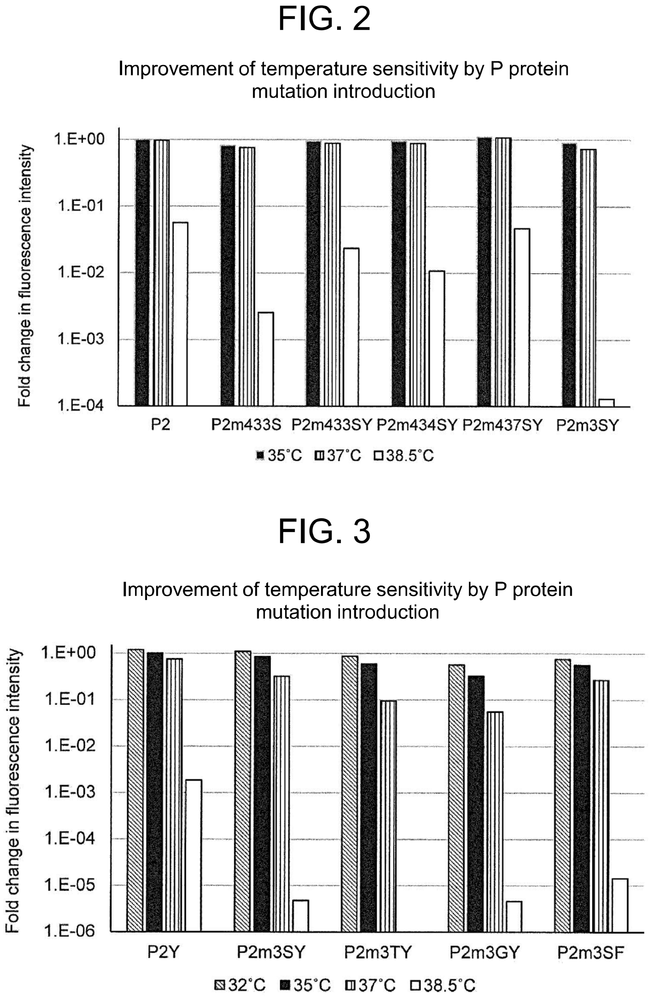

Next, the Vero cell was infected with the resulting SEV mutant (MOI = 20). The infected cell was cultured under the temperature conditions of 35°C or 38.5°C for three days, and the amount of SeV in the infected cell was estimated by measuring the fluorescence from the cell in the same manner as described above. The amount of each fluorescence was determined from the relative value, taking as 1 the amount of fluorescence from the culture of P2 under the temperature conditions of 35°C. The results were as shown in Figures 2 and Table 1. As shown in Figure 2, each mutant exhibited some temperature sensitivity even at a temperature of 37°C, and when it was cultured under the temperature conditions of 38.5°C, the amount of fluorescence decreased significantly. Comparison of relative fluorescence between the mutants in Table 1 clearly shows that each of the mutants was improved in temperature sensitivity compared to P2. Among the mutants, P2m433S showed the greatest increase in temperature sensitivity.

**[Table 1]**

| Table 1: Relative fluorescence intensities of various mutants at each temperature | | | |
|---|---|---|---|
| | 35°C | 37°C | 38.5°C |
| P2 | 100.0% | 96.5% | 5.65% |
| P2m433S | 81.8% | 76.2% | 0.255% |
| P2m433SY | 94.3% | 89.1% | 2.35% |
| P2m434SY | 94.2% | 88.5% | 1.08% |
| P2m437SY | 109.8% | 107.9% | 4.65% |
| P2m3SY | 90.4% | 72.8% | 0.0129% |

The Vero cell was infected with the resulting SEV mutant (MOI = 10). The infected cell was cultured under the temperature conditions of 32°C, 35°C, 37°C, or 38.5°C. The SeV vector expresses EmGFP in a cell and the infected cell emits fluorescence depending on the amount of SeV in the cell. Each mutant was cultured under each of the temperature conditions and the amount of fluorescence from the cells was measured after 3 days. The results were as shown in Figures 3 and Table 2. The results were expressed as the relative fluorescence intensity, taking the fluorescence intensity of P2Y as 100%. As shown in Figure 3, each mutant exhibited the temperature sensitivity even at 37°C, and the amount of fluorescence decreased significantly when cultured at 38.5°C. Comparison of relative fluorescences in Table 2 clearly shows that each of the mutants was improved in temperature sensitivity compared to P2.

**[Table 2]**

| Table 2: Relative fluorescence intensities of various mutants at each temperature | | | | |
|---|---|---|---|---|
| | 32°C | 35°C | 37°C | 38.5°C |
| P2Y | 118.9% | 100.0% | 74.6% | 0.186% |
| P2m3SY | 109.2% | 84.8% | 31.9% | 0.00047% |
| P2m3TY | 87.6% | 59.3% | 9.5% | ND |
| P2m3GY | 56.6% | 32.9% | 5.6% | 0.00046% |
| P2m3SF | 75.1% | 56.4% | 26.7% | 0.00144% |

Mutations to an L protein having the amino acid sequence set forth in SEQ ID NO: 5 on the virus genome were further added to each of the above mutants. Mutations were introduced into N1197, L1361, L1558 and K1796 of the L protein. The specific mutations are as follows.
LmCI: N1197S, L1361C, L1558I, K1796E (SEQ ID NO: 17).

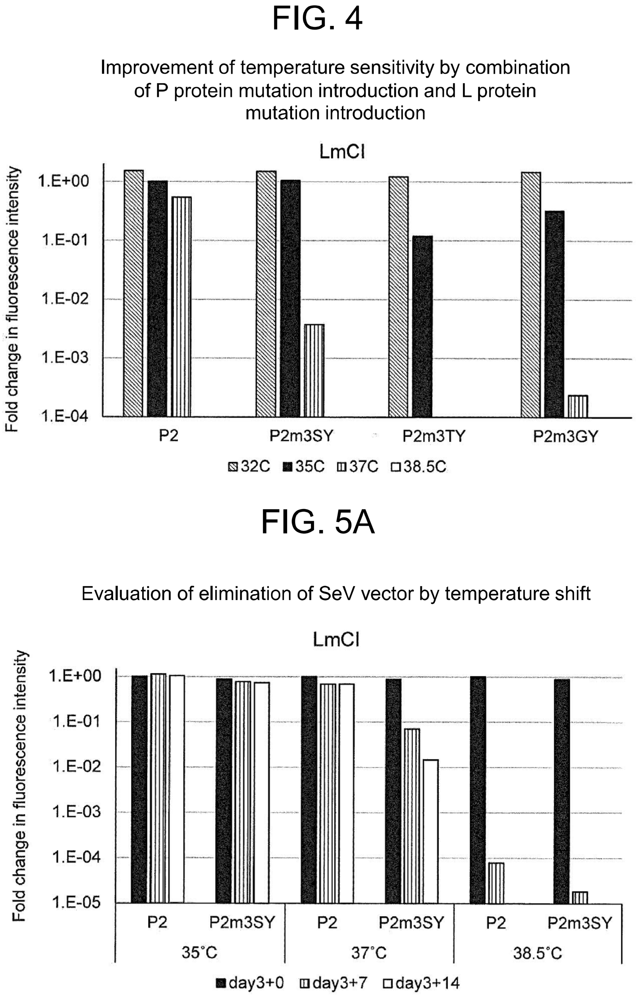

The Vero cell was infected with the SEV obtained from the packaging cell (MOI = 20). The infected cell was then cultured under the temperature conditions of 35°C, 37°C or 38.5°C. The SeV vector expresses EmGFP in a cell and the infected cell emits fluorescence depending on the amount of SeV in the cell. Each mutant was cultured under each of the temperature conditions and the amount of fluorescence from the cells was measured after 3 days. The results were as shown in Figure 4 and Table 3. The results were expressed as the relative fluorescence intensity, taking the fluorescence intensity of P2 + LmCI as 100%. As a result, for each of P2m3SY + LmCI, P2m3TY + LmCI, and P2m3GY + LmCI, the amount of fluorescence decreased after culturing it at 37°C and was not detected after culturing it at 38.5°C. Each of P2m3TY + LmCI and P2m3GY + LmCI exhibited a strong temperature sensitivity even when cultured under the temperature conditions of 35°C and 37°C. Even when the mutant having the amino acid sequence set forth in SEQ ID NO: 7 as an L protein was used, the temperature sensitivity was exhibited, but the mutant having the amino acid sequence set forth in SEQ ID NO: 17 was higher in temperature sensitivity. This indicates that the L protein can give the temperature sensitivity to SeV by introducing one or both of a mutation to L1361 and a mutation to L1558.

**[Table 3]**

| Table 3: Relative fluorescence intensities of various mutants at each temperature | | | | |
|---|---|---|---|---|
| | 32°C | 35°C | 37°C | 38.5°C |
| P2 | 151.8% | 100.0% | 54.6% | ND |
| P2m3SY | 150.0% | 104.3% | 0.38% | ND |
| P2m3TY | 122.0% | 12.0% | ND | ND |
| P2m3GY | 147.6% | 32.5% | 0.024% | ND |

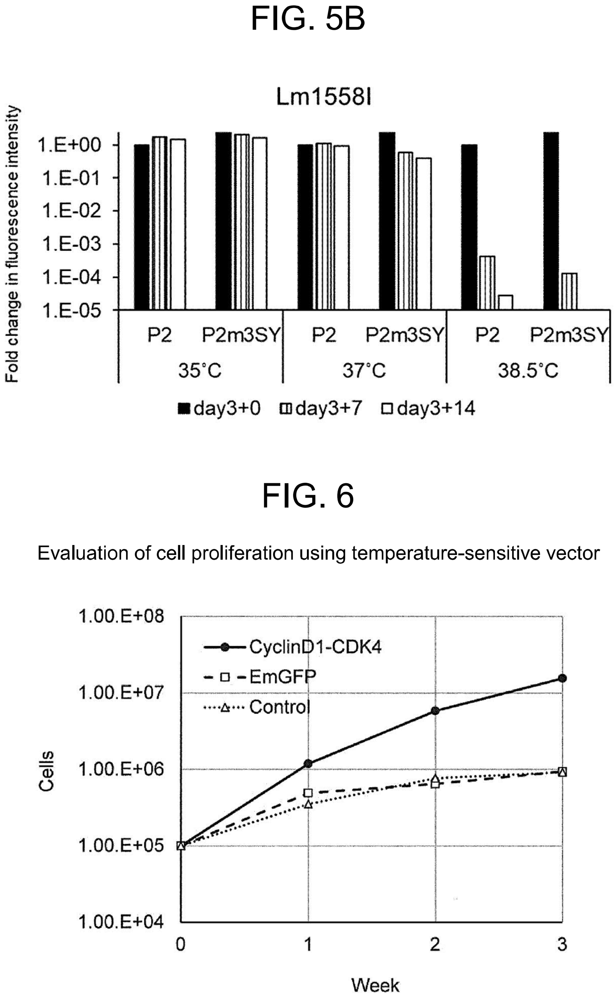

In the same manner, each of various mutants having LmCI was obtained from a packaging cell, and a Vero cell was infected therewith at 35°C (MOI = 20). It was cultured at 35°C for three days, passaged, and thereafter cultured under the temperature conditions of 35°C, 37°C or 38.5°C for another 7 or 14 days. The fluorescence intensity from each cell was measured. The results were as shown in Figures 5 and Table 4. As shown in Figure 5A and Table 4, P2m3SY+LmCI exhibited some temperature sensitivity even under the temperature conditions of 37°C, and exhibited a strong temperature sensitivity under the conditions of temperature of 38.5°C.

**[Table 4]**

| Table 4: Relative fluorescence intensities of various mutants at each temperature | | | | |
|---|---|---|---|---|
| | | day3+0 | day3+7 | day3+14 |
| 35°C | P2 | 100% | 112.57% | 103.62% |
| | P2m3SY | 88% | 76.72% | 73.34% |
| 37°C | P2 | 100% | 68.70% | 68.98% |
| | P2m3SY | 88% | 7.04% | 1.47% |
| 38.5°C | P2 | 100% | 0.0078% | ND |
| | P2m3SY | 88% | 0.0018% | ND |

In addition, each of various mutants having Lm1558I was obtained from a packaging cell, and the Vero cell was infected therewith at 35°C (MOI = 20). It was cultured at 35°C for three days, passaged, and thereafter cultured under the temperature conditions of 37°C or 38.5°C for another 7 or 14 days. The fluorescence intensity from each cell was measured. The results were as shown in Figures 5B and Table 4-1. As shown in Figures 5B and Table 4-1, P2m3SY+Lm1558I exhibited some temperature sensitivity even under the temperature conditions of 37°C, but exhibited a strong temperature sensitivity under the conditions of temperature of 38.5°C.

**[Table 4-1]**

| | | day3+0 | day3+7 | day3+14 |
|---|---|---|---|---|
| 35°C | P2 | 100% | 169% | 144% |
| | P2m3SY | 235% | 201% | 164% |
| 37°C | P2 | 100% | 108% | 91.3% |
| | P2m3SY | 235% | 57.5% | 38.7% |
| 38.5°C | P2 | 100% | 0.042% | 0.0028% |
| | P2m3SY | 235% | 0.013% | ND |

### (5) Introduction of gene of interest into cell by SeV

A human fibroblast was infected with a SeV loaded with proliferation factors, and the number of cells was counted. P2m3SY was used as the SeV, and its genome was loaded with cyclin D1 and CDK4 (R24C) as proliferation factors. The human fibroblasts were infected with the proliferation factors-loaded SeV vector as a test vector and with the EmGFP-loaded SeV vector as a negative control (MOI=20) and passaged every 7 days, and the number of cells was counted. The results were as shown in Figure 6. The cell number increased to 156 times the original number of cells in the proliferation factors-loaded SeV vector. In contrast, the number of cells in the negative control stayed only 9 times the original number of cells. Thus, the temperature-sensitive vector of the present invention is suitable for introducing a foreign gene into an infected cell and expressing it in the infected cell. In addition, after culturing the cell, raising the temperature to a higher temperature can eliminate the SeV vector and reduces the level of vectors in cells to the detection limit or less. This clearly shows that the SeV vector system capable of being eliminated could be constructed after gene transfer.

In addition, in the present Example, a combination of CyclinD1, CDK4 (R24C), and TERT (the above combination is hereinafter referred to as "CCT") was introduced into a human umbilical cord matrix-derived mesenchymal stem cell (MSC) and a human neonatal foreskin-derived fibroblast (an NHDF cell) by the above-described temperature-sensitive vector (SeV/TSdF-P2m434SY), and its effect on cell proliferation was checked. The Sendai virus genome was loaded with CCT at the position PM and mEmerald at the position MHN. The cells were cultured in DMEM/F12 medium containing 10% FBS on a collagen plate. The cells were passaged every 7 days, and the number of cells was counted at each passage. At each passage, a fixed number of cells were collected and seeded in a fixed volume of fresh medium. After 42 days, the number of cells was measured and the proliferation rate was calculated. The results were as shown in Table 5. As shown in Table 5, the MSCs of the control proliferated up to 137 times, whereas the MSCs having CCT introduced thereinto using the SeV of the present invention proliferated up to 13.2 million times. On the other hand, in 42 days, the NHDF cells of the control proliferated up to 12.1 times, whereas the NHDF cells having CCT introduced thereinto using the SeV of the present invention proliferated up to 1.47 million times.

**[Table 5]**

| Table 5: Effect of CCT introduction on proliferation of MSCs and NHDF cells | | | | | |
|---|---|---|---|---|---|
| Cell proliferation of MSCs (35°C) | | | Cell proliferation of NHDF cells (35°C) | | |
| | day0 | day42 | | day0 | day42 |
| Control | 1.00.E+05 | 1.37.E+07 | Control | 1.00E+05 | 1.21.E+06 |
| CCT | 1.00.E+05 | 1.32.E+12 | CCT | 1.00E+05 | 1.47.E+11 |

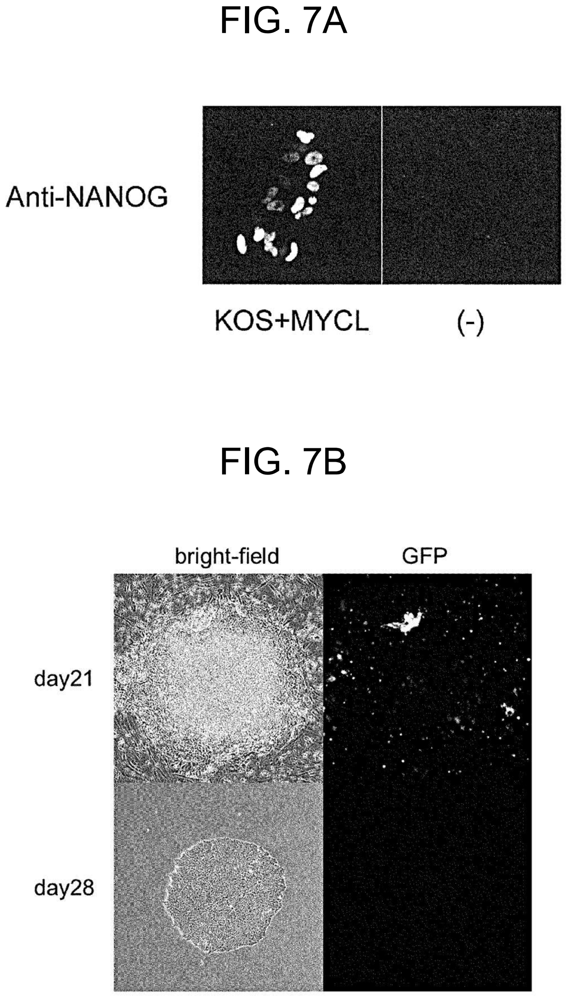

In addition, in the present Example, a human fibroblast was infected with a Sendai virus vector loaded with a reprogramming factor (MOI= 20), and the cell after infection was observed by immunocytochemical staining using an anti-NANOG antibody. The reprogramming factor used was KLF4, OCT4, SOX2 and MYCL. The vector was a vector having P2m3SY and LmCI mutations, and one simultaneously loaded with KLF4, OCT4, and SOX2 (KOS) and one loaded with MYCL were prepared. The human fibroblast was infected with two types of vectors and was passaged after five days, and the culture medium was replaced by a StemFit medium after six days. The expression of NANOG in the cell after 11 days was observed using the anti-NANOG antibody. Then, as shown in Figure 7A, the human fibroblast treated with the Sendai virus vector was reprogrammed and NANOG was induced. If the temperature of the culture medium is increased to 38.5°C after reprogramming the cell, the Sendai virus vector will be eliminated from the cell and the level of vectors in cells will be reduced to the detection limit or less. However, when the fibroblast having the reprogramming factor(s) introduced thereinto was cultured in a StemFit AK92N medium on a plate coated with iMatrix-511, the SeV vector was gradually eliminated from the cell even under the temperature conditions of 37°C, and the fluorescence of EmGFP expressed from the SeV was reduced to the detection limit or less after 28 days (see Figure 7B).

### (6) Promotion of further elimination of SeV by degron

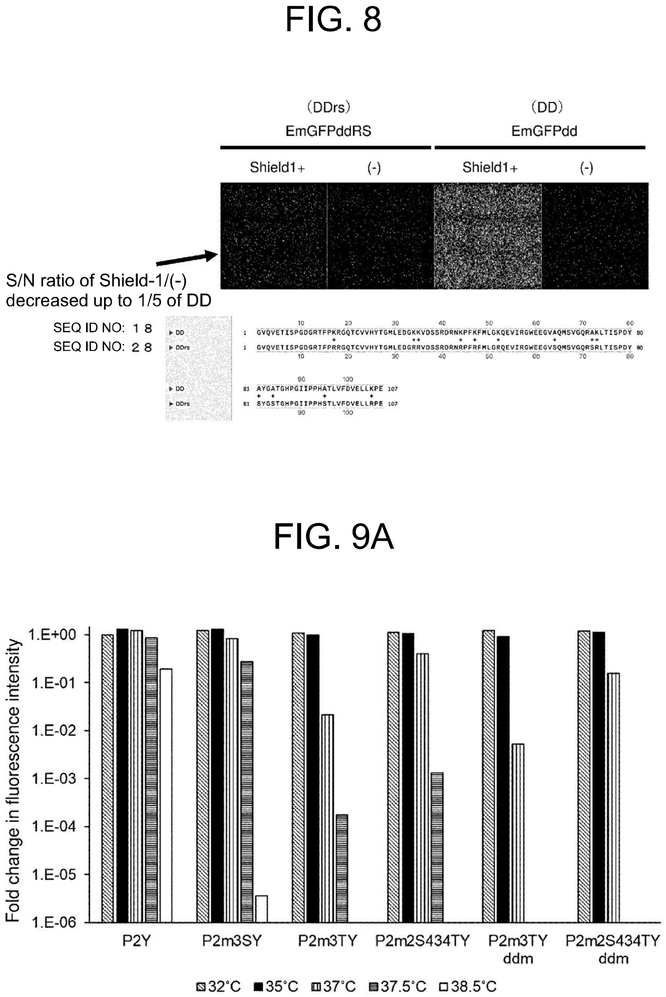

The addition of a proteolytic tag (degron) to a P protein can facilitate the elimination of a SeV. A DD tag is a type of proteolytic tag (see, for example, US 2009/0215169 A). The DD tag (see SEQ ID NO: 18) is a protein of 107 amino acids in length, obtained by introducing F36V and L106P into FKBP12. Adding a low molecular weight compound, Shield-1 provides stabilization, and adding dTAG-13 also promotes degradation of the protein fused with the tag. In the present Example, a DD mutant (DDm; see SEQ ID NO: 19) having 11 mutations (4I, 18K, 36L, 50I, 57K, 59F, 63A, 68L, 90V, 98I, and 105R) in a DD tag was constructed. In the present Example, in order to investigate the proteolysis-promoting effect of the DDm, the DD tag and its mutants (including the DDm) were added to EmGFP at its C-terminal and loaded at the + position of the SeV vector (having the above mutation described in WO 2003/025570). HeLa cells were infected with the resulting SeV (MOI = 10). On the day after infection, 1 µM of Shield-1 or 5 µM of dTAG-13 was added thereto, and the fluorescence from the cell was observed 3 days after infection. The results were as shown in Tables 6 and 7. When a DDrs tag having the amino acid sequence set forth in SEQ ID NO: 28 was constructed and added to EmGFP at its C-terminal, the effect of stabilizing EmGFP by Sheild-1 was not obtained and the fluorescence intensity was only about 20% of that of a DD-tagged EmGFP (Figure 8).

**[Table 6]**

| Table 6: Degradation promoting effect by DDm tag | | | | | |
|---|---|---|---|---|---|
| Relative fluorescence intensity of EmGFP having degron added thereto (37°C) | | | | | |
| 37°C | Shield1 | dTAG-13 | (-) | Shield1/(-) | Shield1/ dTAG-13 |
| DD | 100.0% | 0.545% | 25.2% | 3.97 | 183.3 |
| DD36I | 109.5% | 0.602% | 26.6% | 4.11 | 181.9 |
| DD36L | 129.8% | 1.098% | 29.8% | 4.35 | 118.2 |
| DD36L90V | 144.4% | 1.301% | 50.4% | 2.87 | 111.0 |
| DDm | 140.4% | 0.478% | 15.0% | 9.39 | 293.9 |

**[Table 7]**

| Table 7: Degradation promoting effect by DDm tag | | | | | |
|---|---|---|---|---|---|
| Relative fluorescence intensity of EmGFP having degron added thereto (38.5°C) | | | | | |
| 38.5°C | Shield1 | dTAG-13 | (-) | Shield1/(-) | Shield1/ dTAG-13 |
| DD | 100% | 0.10% | 7.3% | 13.79 | 973.3 |
| DD36I | 137% | 0.10% | 8.0% | 17.21 | 1328.0 |
| DD36L | 167% | 0.16% | 12.5% | 13.36 | 1025.4 |
| DD36L90V | 191% | 0.18% | 22.5% | 8.48 | 1085.2 |
| DDm | 153% | 0.05% | 2.5% | 61.47 | 3181.7 |

As shown in Tables 6 and 7, the DDm was improved in the stability to Shield-1, and improved the degradation promoting effect by dTAG-13. As a result, the ratio of Shield1/dTAG-13 was significantly improved.

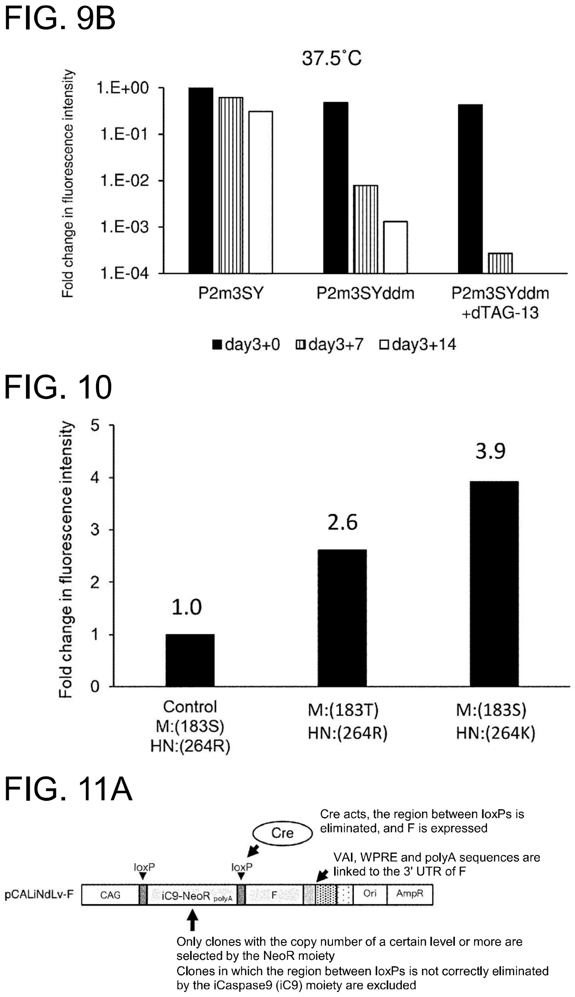

Next, a DDm tag was added to each of various temperature-sensitive mutant of a P protein at its C-terminal. EmGFP was loaded at the + position of the SeV genome. A HeLa cell was infected with the SeV expressing each of various temperature-sensitive mutant having the DDm tag added thereto (MOI = 10), and the fluorescence intensity derived EmGFP was checked after three days. The results were as shown in Figure 9 and Table 8.

**[Table 8]**

| Table 8: Elimination of SeV having P protein having DDm introduced or not introduced thereinto from cell | | | | | |
|---|---|---|---|---|---|
| | 32°C | 35°C | 37°C | 37.5°C | 38.5°C |
| P2Y | 100.0% | 131.2% | 122.5% | 87.0% | 19.1% |
| P2m3SY | 121.6% | 128.4% | 83.4% | 27.6% | 0.00036% |
| P2m3TY | 109.9% | 100.1% | 2.1% | 0.018% | N.D. |
| P2m2S434TY | 110.8% | 107.2% | 40.2% | 0.132% | N.D. |
| P2m3TYddm | 121.7% | 92.4% | 0.5% | N.D. | N.D. |
| P2m2S434TYddm | 118.9% | 111.4% | 15.8% | N.D. | N.D. |

| | | | | | |
|---|---|---|---|---|---|
| 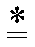 N.D. indicates that the fluorescence was at or below the detection limit. | | | | | |

As shown in Figure 9A and Table 8, both of P2m3TYddm and P2m2S434TYddm having DDm added thereto improved the efficiency of eliminating SeV and promoted the elimination of SeV from a cell even under the temperature conditions of 37.5°C. The results are those obtained in the absence of dTAG-13, suggesting that the addition of the DDm tag destabilizes the P protein even in the absence of dTAG-13. As shown in Figure 9B and Table 9, the destabilization of P proteins having the DDm tag added thereto was further promoted in the presence of dTAG-13.

**[Table 9]**

| Table 9: Protein stabilizing effect by DDrs tag | | | | |
|---|---|---|---|---|
| | | day3+0 | day3+7 | day3+14 |
| | P2m3SY | 100.0% | 61.5% | 30.8% |
| 37.5°C | P2m3SYddm | 47.9% | 0.787% | 0.13% |
| | P2m3SYddm+dTAG-13 | 42.3% | 0.027% | N.D. |

### (7) Reduction in cytotoxicity of SeV

SeV induces cytotoxicity. For the purpose of reducing this cytotoxicity, an amino acid mutation(s) can be induced into an M protein and an HN protein. As described above, the SeV of the present invention have basically already had such a mutations(s) introduced thereinto (WO 2003/025570). In the present Example, the amino acid mutation(s) in each of the M protein and HN protein were further investigated. Specifically, A183T was introduced into the M protein in addition to G69E and T116A (see SEQ ID NO: 20). On the other hand, G264K was introduced into the HN protein in addition to A262T and K461E (see SEQ ID NO: 21). L511Y was introduced into a P protein, and N1197S and K1796E was introduced into an L protein. Then, an SeV having EmGFP at the + position thereof was constructed and Vero cells was infect with the SeV, and the infected cell cultured at 32°C for 20 days. The fluorescence intensity derived from EmGFP in the Vero cells was measured.

The results were as shown in Figure 10. As shown in Figure 10, the M protein having A183T increased the fluorescence intensity to 2.6 times that of the control, and the HN protein having G264K increased the fluorescence intensity to 3.9 times that of the control. The detachment of the EmGFP-positive cell from the dish observed in the control was observed and the detachment was reduced in the SeV-infected cell having the mutants described above. Therefore, it was suggested that the mutations reduced the cytotoxicity induced by the SeV. When the SeV vector having the above mutant of the M protein was loaded with a reprogramming factor and a fibroblast was infected with the SeV vector, reprogramming of the fibroblast could be induced.

### (8) Construction of packaging cell

A packaging cell for a negative-strand RNA virus or virus vector was established from each of a Vero cell and an LLC-MK2 cell. Specifically, a vector was loaded with a gene encoding F and a gene encoding a PKR inhibitory factor, and introduced into a packaging cell to obtain, as a stable cell line, a packaging cell that expresses the gene encoding F and the gene encoding a PKR inhibitory factor. VAI was used as the PKR inhibitory factor. The insertion position of VAI is not particularly limited, but in the present Example, the sequence encoding VAI was introduced downstream of the gene encoding F (see Figure 11A). These loaded genes can be driven by any promoter, but in the present example, they were driven by a CAG promoter (see Figure 11A). However, a region flanked by two site-specific recombinant sequences (in this Figure, loxPs) was caused to intervene between the CAG promoter and the gene encoding F, the region flanked between the two loxPs was eliminated by a Cre recombinase or the like to construct a vector so that the gene encoding F was driven more strongly by the CAG promoter. A gene encoding a fusion protein having iCaspase9 and NeoR (a neomycin resistance gene) linked by a 2A sequence was loaded between the two loxPs. This enabled the selection of a clone that is high in expression level of the NeoR to the extent that it can survive in the presence of a drug such as G418 at a certain concentration (including a clone that is large in the number of introduced copies). Based on such as design concept, a pCALiNdLv-F vector was constructed from a pCALNdL vector (JOURNAL OF VIROLOGY, Feb. 1998, p.1115-1121) (Figure 11A). SeV-Cre was loaded with a gene encoding a Cre recombinase at the + position and loaded with mEmerald, a fluorescent protein, at the MHN position. This allows proliferation of the virus to be confirmed by the fluorescence of the mEmerald. SeV-Cre can also have a temperature-sensitive genome that is eliminated from a cell in a temperature-dependent manner.

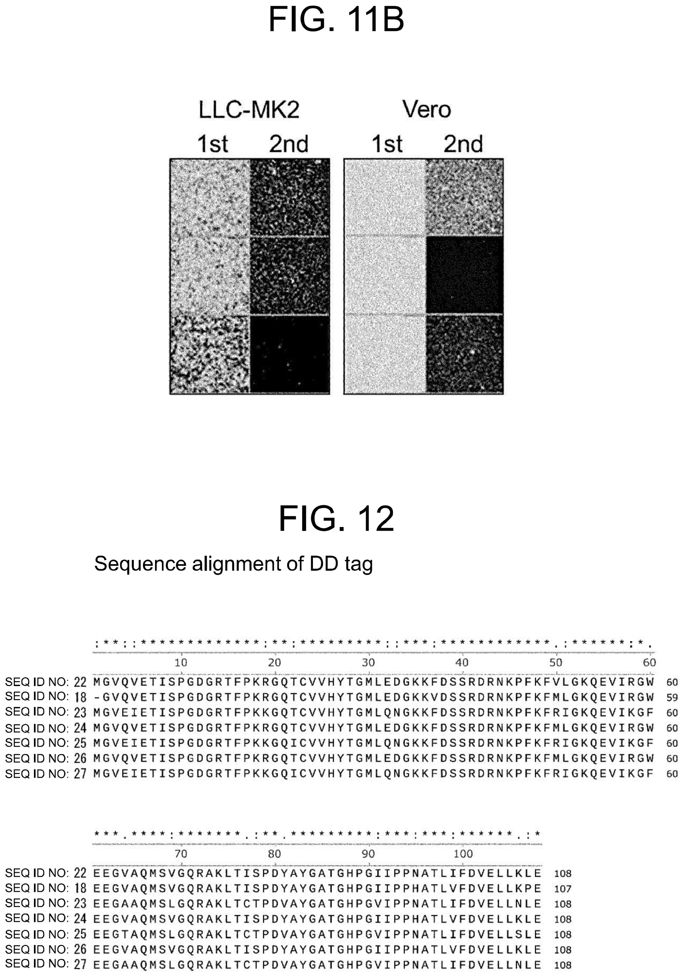

The pCALiNdLv-F vector was introduced into each of the LLC-MK2 cell and the Vero cell, and thereafter subjected to drug selection with G418 at 37°C. SeV-Cre was introduced into the resulting clone and the cell was cultured at 32°C. As a result, it well infected both of the LLC-MK2 cell and the Vero cell, as shown in Figure 11B (1st). The supernatant from the 1st cell was contacted with the 2nd cell. If the supernatant contains a virus with infectivity, it should infect the 2nd cell and cause the cell to emit the fluorescence derived from the mEmerald. The results of the infection experiment for the 2nd cell showed that the supernatant infected both the LLC-MK2 cell and the Vero cell and caused each cell to emit the fluorescence derived from the mEmerald (see the 2nd cell in Figure 11B). If the supernatant does not contain a virus with infectivity, the virus does not infect the 2nd cell and cannot cause each cell to emit the fluorescence derived from the mEmerald, as shown in the lower panel in Figure 11B (2nd) for the LLC-MK2 cell and in the middle panel in Figure 11B (2nd) for the Vero cell. In the present example, the virus with infectivity was able to be reproducibly produced in both the LLC-MK2 cell and the Vero cell. Therefore, it can be understood by those skilled in the art that the virus with infectivity could be produced as appropriate. More particularly, the above results suggest that clones highly expressing F and PKR inhibitory factors could be selected by drug selection with G418 in the LLC-MK2 cell and the Vero cell (1.3 to 2.5 mg/mL of G418 was used for the LLC-MK2 cell and 0.9-1.5 mg/mL of G418 was used for the Vero cell), and that the Cre recombinase loaded on SeV acted to eliminate the sequence between the two loxPs, resulting in high expression of F. As a result, SeV was observed to be produced in the 1st cell and it also infected the 2nd cell, clearly indicating that a virus with infectivity could be produced in the 1st cell. Thus, the above-described plasmid was suggested to be a useful tool for constructing packaging cells from the 1st cell (conventional LLC-MK2 cell and Vero cell). In the present system, SeV-Cre is inferred to have been left in the 1st cell. Therefore, by subsequently eliminating SeV-Cre in the 1st cell, the resulting cell can be utilized as a packaging cell for SeV reconstitution. SeV-Cre can be eliminated by simply passaging cells, but various methods are known to increase elimination efficiency such as utilization of a temperature sensitive cell line or utilization of an microRNA and may be used as appropriate to eliminate the SeV-Cre. When the cell was subsequently subjected to further re-cloning under the temperature conditions of 37°C, the F-expressing cell having the SeV eliminated therefrom was obtained. F expression enabled LLC-MK2 and Vero cells to be used as a packaging cell, but it can be understood that F may be supplied from a cell side, by a plasmid, or from Sendai virus genome.

### (9) Virus removal by bromotag

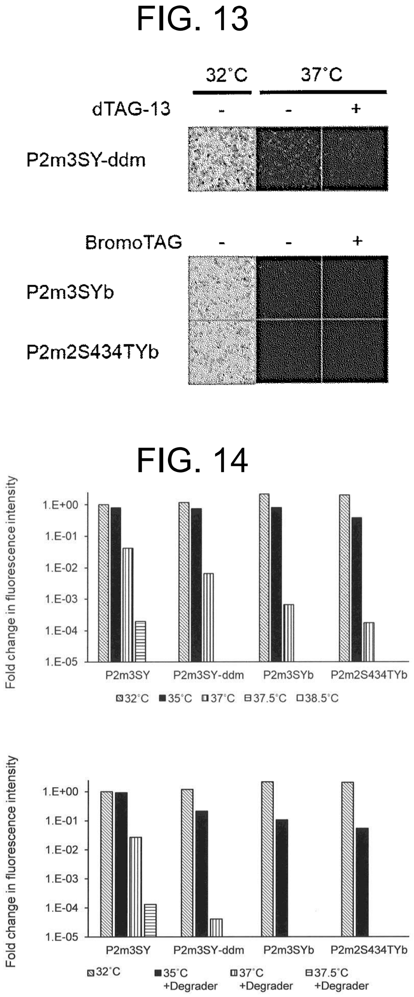

HeLa cells were infected at 32-38.5°C with SeV (MOI = 20) having proteins in which Brd4^{BD2 (L387A)} was added to the C-terminus of modified P proteins (P2m3SY, P2m2S434TY, and P2m3TY) (respectively, P2m3SYb, P2m2S434TYb, and P2m3Tb). As a control, HeLa cells were infected under the same conditions with SeV (MOI = 20) having a protein in which ddm was added to the modified P protein (P2m3SY) (P2m3SYddm). On the day after SeV infection, 5 µM dTAG-13 or 1 µM BromoTAG (trade name) AGB1 was added as a ligand (degrader) to induce degradation of the modified P proteins, and fluorescence of EmGFP was evaluated on the second day after SeV infection. AGB1 recruits von Hippel-Lindau (VHL) E3 ligase to Brd4^{BD2 (L387A)} and promotes degradation of the Brd4^{BD2 (L387A)} -fused proteins. AGB1 is (S)-13-((2S,4R)-4-hydroxy-2-(((4-(4-methylthiazol-5-yl)benzyl)carbamoyl)pyrrolidin-1-carbonyl))-14,14-dimethyl-11-oxo-3,6,9-trioxa-12-azapentadecyl (R)-2-(((S)-4-(4-chlorophenyl)-2,3,9-trimethyl-6H-thieno[3,2-f][1,2,4]triazolo[4,3-a][1,4]diazepin-6-yl)butanoate. As shown in Figure 13, the combination of Brd4BD2(L387A) and BromoTAG promoted SeV removal more effectively than the combination of ddm and dTAG-13.

Similar experiments were conducted using various P protein variants. The results were as shown in Figure 14 and in Tables 10 and 11. Tables 10 and 11 summarize the results of Figure 14.

**[Table 10]**

| Table 10: SeV removal effect by bromotag, ddm tag, and temperature-sensitive P protein in the absence of a ligand (Degrader) for the tag | | | | | |
|---|---|---|---|---|---|
| | 32°C | 35°C | 37°C | 37.5°C | 38.5°C |
| P2m3SY | 100.0% | 80.5% | 4.23% | 0.019% | 0.000% |
| P2m3SY-ddm | 119.0% | 76.5% | 0.65% | 0.000% | 0.000% |
| P2m3SYb | 215.9% | 79.5% | 0.07% | 0.000% | 0.000% |
| P2m2S434TYb | 205.3% | 38.5% | 0.02% | 0.000% | 0.000% |

**[Table 11]**

| Table 11: SeV removal effect by bromotag, ddm tag, and temperature-sensitive P protein in the presence of a ligand (Degrader) for the tag | | | | |
|---|---|---|---|---|
| | 32°C | 35°C +Degrader | 37 C +Degrader | 37.5°C +Degrader |
| P2m3SY | 100.0% | 92.1% | 2.698% | 0.013% |
| P2m3SY-ddm | 119.0% | 21.3% | 0.004% | 0.000% |
| P2m3SYb | 215.9% | 10.6% | 0.000% | 0.000% |
| P2m2S434TYb | 205.3% | 5.35% | 0.000% | 0.000% |

As shown in Figure 14 and Table 10, in the absence of a ligand (Degrader) for the tag, SeV containing all P protein variants was detected at 37°C. However, in the presence of the ligand, SeV containing P proteins fused with a bromotag was below the detection limit. Furthermore, it was found that the SeV removal effect was higher than that with ddm.

Next, the bromotag was utilized for controlling expression of a carried gene. SeV (MOI = 10) carrying EmGFP with Brd4BD2(L387A) fused (referred to as EmGFPb) was infected into HeLa cells at 37°C. In addition, SeV+EmGFPb/TSdF was infected into HeLa cells at MOI = 10. On the day after SeV infection, 1 µM BromoTAG (trade name) AGB1 was added, and fluorescence of EmGFPb was evaluated on the second day after SeV infection. The results are shown in Table 12. As shown in Table 12, the fluorescence of EmGFPb was reduced to 2.3% of that without addition upon addition of AGB1.

### [Table 12]

**Table 12: Effect of bromotag on protein expression**

| | | |
|---|---|---|
| | - | BromoTAG |
| EmGFPb | 100.0% | 2.3% |

From the above, in the present disclosure, it was possible to achieve removal of SeV and control of expression (degradation) of specific proteins by using a bromotag and a PROTAC against it.

### Sequence listing:

SEQ ID NO: 1: an example of the amino acid sequences of the wild-type P protein of Sendai virus 
TNSVSQDLGD GGDSGILENP PNERGYPRSG IEDENREMAA HPDKRGEDQA EGLPEEVRGS 180

TSLPDEGEGG ASNNGRSMEP GSSHSARVTG VLVIPSPELE EAVLRRNKRR PTNSGSKPLT 240

PATVPGTRSP PLNRYNSTGS PPGKPPSTQD EHINSGDTPA VRVKDRKPPI GTRSVSDCPA 300
 
NGRPIHPGLE TDSTKKGIGE NTSSMKEMAT LLTSLGVIQS AQEFESSRDA SYVFARRALK 360

SANYAEMTFN VCGLILSAEK SSARKVDENK QLLKQIQESV ESFRDIYKRF SEYQKEQNSL 420

LMSNLSTLHI ITDRGGKTDN TDSLTRSPSV FAKSKENKTK ATRFDPSMET LEDMKYKPDL 480

IREDEFRDEI RNPVYQERDT EPRASNASRL LPSKEKPTMH SLRLVIESSP LSRAEKVAYV 540

KSLSKCKTDQ EVKAVMELVE EDIESLTN 568
SEQ ID NO: 2: an amino acid sequence of the P protein mutant (P2) of Sendai virus 
MDQDAFILKE DSEVEREAPG GRESLSDVIG FLDAVLSSEP TDIGGDRSWL HNTINTPQGP 60

GSAHRAKSEG EGEVSTPSTQ DNRSGEESRV SGRTSKPEAE AHAGNLDKQN IHRAFGGRTG 120

TNSVSQDLGD GGDSGILENP PNERGYPRSG IEDENREMAA HPDKRGEDQA EGLPEEVRGS 180

TSLPDEGEGG ASNNGRSMEP GSSHSARVTG VLVIPSPELE EAVLRRNKRR PTNSGSKPLT 240

PATVPGTRSP PLNRYNSTGS PPGKPPSTQD EHINSGDTPA VRVKDRKPPI GTRSVSDCPA 300

NGRPIHPGLE TDSTKKGIGE NTSSMKEMAT LLTSLGVIQS AQEFESSRDA SYVFARRALK 360

SANYAEMTFN VCGLILSAEK SSARKVDENK QLLKQIQESV ESFRDIYKRF SEYQKEQNSL 420

LMSNLSTLHI ITAAGGATDN TDSLTRSPSV FAKSKENKTK ATRFDPSMET LEDMKYKPDL 480

IREDEFRDEI RNPVYQERDT EPRASNASRL FPSKEKPTMH SLRLVIESSP LSRAEKVAYV 540

KSLSKCKTDQ EVKAVMELVE EDIESLTN 568
SEQ ID NO: 3: an amino acid sequence of the P protein mutant (L551F) of Sendai virus 
MDQDAFILKE DSEVEREAPG GRESLSDVIG FLDAVLSSEP TDIGGDRSWL HNTINTPQGP 60

GSAHRAKSEG EGEVSTPSTQ DNRSGEESRV SGRTSKPEAE AHAGNLDKQN IHRAFGGRTG 120

TNSVSQDLGD GGDSGILENP PNERGYPRSG IEDENREMAA HPDKRGEDQA EGLPEEVRGS 180

TSLPDEGEGG ASNNGRSMEP GSSHSARVTG VLVIPSPELE EAVLRRNKRR PTNSGSKPLT 240

PATVPGTRSP PLNRYNSTGS PPGKPPSTQD EHINSGDTPA VRVKDRKPPI GTRSVSDCPA 300

NGRPIHPGLE TDSTKKGIGE NTSSMKEMAT LLTSLGVIQS AQEFESSRDA SYVFARRALK 360

SANYAEMTFN VCGLILSAEK SSARKVDENK QLLKQIQESV ESFRDIYKRF SEYQKEQNSL 420

LMSNLSTLHI ITDRGGKTDN TDSLTRSPSV FAKSKENKTK ATRFDPSMET LEDMKYKPDL 480

IREDEFRDEI RNPVYQERDT EPRASNASRL FPSKEKPTMH SLRLVIESSP LSRAEKVAYV 540
 KSLSKCKTDQ EVKAVMELVE EDIESLTN 568
SEQ ID NO: 4: an amino acid sequence of a temperature-sensitive P protein mutant of Sendai virus 
MDQDAFILKE DSEVEREAPG GRESLSDVIG FLDAVLSSEP TDIGGDRSWL HNTINTPQGP 60

GSAHRAKSEG EGEVSTPSTQ DNRSGEESRV SGRTSKPEAE AHAGNLDKQN IHRAFGGRTG 120

TNSVSQDLGD GGDSGILENP PNERGYPRSG IEDENREMAA HPDKRGEDQA EGLPEEVRGS 180

TSLPDEGEGG ASNNGRSMEP GSSHSARVTG VLVIPSPELE EAVLRRNKRR PTNSGSKPLT 240

PATVPGTRSP PLNRYNSTGS PPGKPPSTQD EHINSGDTPA VRVKDRKPPI GTRSVSDCPA 300

NGRPIHPGLE TDSTKKGIGE NTSSMKEMAT LLTSLGVIQS AQEFESSRDA SYVFARRALK 360

SANYAEMTFN VCGLILSAEK SSARKVDENK QLLKQIQESV ESFRDIYKRF SEYQKEQNSL 420

LMSNLSTLHI ITXXGGXTDN TDSLTRSPSV FAKSKENKTK ATRFDPSMET LEDMKYKPDL 480

IREDEFRDEI RNPVYQERDT EPRASNASRL XPSKEKPTMH SLRLVIESSP LSRAEKVAYV 540

KSLSKCKTDQ EVKAVMELVE EDIESLTN 568
SEQ ID NO: 5: an example of the amino acid sequences of the wild-type L protein of Sendai virus 
MDGQESSQNP SDILYPECHL NSPIVRGKIA QLHVLLDVNQ PYRLKDDSII NITKHKIRNG 60

GLSPRQIKIR SLGKALQRTI KDLDRYTFEP YPTYSQELLR LDIPEICDKI RSVFAVSDRL 120

TRELSSGFQD LWLNIFKQLG NIEGREGYDP LQDIGTIPEI TDKYSRNRWY RPFLTWFSIK 180

YDMRWMQKTR PGGPLDTSNS HNLLECKSYT LVTYGDLVMI LNKLTLTGYI LTPELVLMYC 240

DVVEGRWNMS AAGHLDKKSI GITSKGEELW ELVDSLFSSL GEEIYNVIAL LEPLSLALIQ 300

LNDPVIPLRG AFMRHVLTEL QTVLTSRDVY TDAEADTIVE SLLAIFHGTS IDEKAEIFSF 360

FRTFGHPSLE AVTAADKVRA HMYAQKAIKL KTLYECHAVF CTIIINGYRE RHGGQWPPCD 420

FPDHVCLELR NAQGSNTAIS YECAVDNYTS FIGFKFRKFI EPQLDEDLTI YMKDKALSPR 480

KEAWDSVYPD SNLYYKAPES EETRRLIEVF INDENFNPEE IINYVESGDW LKDEEFNISY 540

SLKEKEIKQE GRLFAKMTYK MRAVQVLAET LLAKGIGELF SENGMVKGEI DLLKRLTTLS 600

VSGVPRTDSV YNNSKSSEKR NEGMENKNSG GYWDEKKRSR HEFKATDSST DGYETLSCFL 660

TTDLKKYCLN WRFESTALFG QRCNEIFGFK TFFNWMHPVL ERCTIYVGDP YCPVADRMHR 720

QLQDHADSGI FIHNPRGGIE GYCQKLWTLI SISAIHLAAV RVGVRVSAMV QGDNQAIAVT 780
 
SRVPVAQTYK QKKNHVYEEI TKYFGALRHV MFDVGHELKL NETIISSKMF VYSKRIYYDG 840

KILPQCLKAL TKCVFWSETL VDENRSACSN ISTSIAKAIE NGYSPILGYC IALYKTCQQV 900

CISLGMTINP TISPTVRDQY FKGKNWLRCA VLIPANVGGF NYMSTSRCFV RNIGDPAVAA 960

LADLKRFIRA DLLDKQVLYR VMNQEPGDSS FLDWASDPYS CNLPHSQSIT TIIKNITARS 1020

VLQESPNPLL SGLFTETSGE EDLNLASFLM DRKVILPRVA HEILGNSLTG VREAIAGMLD 1080

TTKSLVRASV RKGGLSYGIL RRLVNYDLLQ YETLTRTLRK PVKDNIEYEY MCSVELAVGL 1140

RQKMWIHLTY GRPIHGLETP DPLELLRGIF IEGSEVCKLC RSEGADPIYT WFYLPDNIDL 1200

DTLTNGCPAI RIPYFGSATD ERSEAQLGYV RNLSKPAKAA IRIAMVYTWA YGTDEISWME 1260

AALIAQTRAN LSLENLKLLT PVSTSTNLSH RLKDTATQMK FSSATLVRAS RFITISNDNM 1320

ALKEAGESKD TNLVYQQIML TGLSLFEFNM RYKKGSLGKP LILHLHLNNG CCIMESPQEA 1380

NIPPRSTLDL EITQENNKLI YDPDPLKDVD LELFSKVRDV VHTVDMTYWS DDEVIRATSI 1440

CTAMTIADTM SQLDRDNLKE MIALVNDDDV NSLITEFMVI DVPLFCSTFG GILVNQFAYS 1500

LYGLNIRGRE EIWGHVVRIL KDTSHAVLKV LSNALSHPKI FKRFWNAGVV EPVYGPNLSN 1560

QDKILLALSV CEYSVDLFMH DWQGGVPLEI FICDNDPDVA DMRRSSFLAR HLAYLCSLAE 1620

ISRDGPRLES MNSLERLESL KSYLELTFLD DPVLRYSQLT GLVIKVFPST LTYIRKSSIK 1680

VLRTRGIGVP EVLEDWDPEA DNALLDGIAA EIQQNIPLGH QTRAPFWGLR VSKSQVLRLR 1740

GYKEITRGEI GRSGVGLTLP FDGRYLSHQL RLFGINSTSC LKALELTYLL SPLVDKDKDR 1800

LYLGEGAGAM LSCYDATLGP CINYYNSGVY SCDVNGQREL NIYPAEVALV GKKLNNVTSL 1860

GQRVKVLFNG NPGSTWIGND ECEALIWNEL QNSSIGLVHC DMEGGDHKDD QVVLHEHYSV 1920

IRIAYLVGDR DVVLISKIAP RLGTDWTRQL SLYLRYWDEV NLIVLKTSNP ASTEMYLLSR 1980

HPKSDIIEDS KTVLASLLPL SKEDSIKIEK WILIEKAKAH EWVTRELREG SSSSGMLRPY 2040

HQALQTFGFE PNLYKLSRDF LSTMNIADTH NCMIAFNRVL KDTIFEWARI TESDKRLKLT 2100

GKYDLYPVRD SGKLKTISRR LVLSWISLSM STRLVTGSFP DQKFEARLQL GIVSLSSREI 2160

RNLRVITKTL LDRFEDIIHS ITYRFLTKEI KILMKILGAV KMFGARQNEY TTVIDDGSLG 2220

DIEPYDSS 2228
SEQ ID NO: 6: an amino acid sequence of a temperature-sensitive L protein mutant of Sendai virus MDGQESSQNP SDILYPECHL NSPIVRGKIA QLHVLLDVNQ PYRLKDDSII NITKHKIRNG 60 
GLSPRQIKIR SLGKALQRTI KDLDRYTFEP YPTYSQELLR LDIPEICDKI RSVFAVSDRL 120

TRELSSGFQD LWLNIFKQLG NIEGREGYDP LQDIGTIPEI TDKYSRNRWY RPFLTWFSIK 180

YDMRWMQKTR PGGPLDTSNS HNLLECKSYT LVTYGDLVMI LNKLTLTGYI LTPELVLMYC 240

DVVEGRWNMS AAGHLDKKSI GITSKGEELW ELVDSLFSSL GEEIYNVIAL LEPLSLALIQ 300

LNDPVIPLRG AFMRHVLTEL QTVLTSRDVY TDAEADTIVE SLLAIFHGTS IDEKAEIFSF 360

FRTFGHPSLE AVTAADKVRA HMYAQKAIKL KTLYECHAVF CTIIINGYRE RHGGQWPPCD 420

FPDHVCLELR NAQGSNTAIS YECAVDNYTS FIGFKFRKFI EPQLDEDLTI YMKDKALSPR 480

KEAWDSVYPD SNLYYKAPES EETRRLIEVF INDENFNPEE IINYVESGDW LKDEEFNISY 540

SLKEKEIKQE GRLFAKMTYK MRAVQVLAET LLAKGIGELF SENGMVKGEI DLLKRLTTLS 600

VSGVPRTDSV YNNSKSSEKR NEGMENKNSG GYWDEKKRSR HEFKATDSST DGYETLSCFL 660

TTDLKKYCLN WRFESTALFG QRCNEIFGFK TFFNWMHPVL ERCTIYVGDP YCPVADRMHR 720

QLQDHADSGI FIHNPRGGIE GYCQKLWTLI SISAIHLAAV RVGVRVSAMV QGDNQAIAVT 780

SRVPVAQTYK QKKNHVYEEI TKYFGALRHV MFDVGHELKL NETIISSKMF VYSKRIYYDG 840

KILPQCLKAL TKCVFWSETL VDENRSACSN ISTSIAKAIE NGYSPILGYC IALYKTCQQV 900

CISLGMTINP TISPTVRDQY FKGKNWLRCA VLIPANVGGF NYMSTSRCFV RNIGDPAVAA 960

LADLKRFIRA DLLDKQVLYR VMNQEPGDSS FLDWASDPYS CNLPHSQSIT TIIKNITARS 1020

VLQESPNPLL SGLFTETSGE EDLNLASFLM DRKVILPRVA HEILGNSLTG VREAIAGMLD 1080

TTKSLVRASV RKGGLSYGIL RRLVNYDLLQ YETLTRTLRK PVKDNIEYEY MCSVELAVGL 1140

RQKMWIHLTY GRPIHGLETP DPLELLRGIF IEGSEVCKLC RSEGADPIYT WFYLPDXIDL 1200

DTLTNGCPAI RIPYFGSATD ERSEAQLGYV RNLSKPAKAA IRIAMVYTWA YGTDEISWME 1260

AALIAQTRAN LSLENLKLLT PVSTSTNLSH RLKDTATQMK FSSATLVRAS RFITISNDNM 1320

ALKEAGESKD TNLVYQQIML TGLSLFEFNM RYKKGSLGKP XILHLHLNNG CCIMESPQEA 1380

NIPPRSTLDL EITQENNKLI YDPDPLKDVD LELFSKVRDV VHTVDMTYWS DDEVIRATSI 1440

CTAMTIADTM SQLDRDNLKE MIALVNDDDV NSLITEFMVI DVPLFCSTFG GILVNQFAYS 1500

LYGLNIRGRE EIWGHVVRIL KDTSHAVLKV LSNALSHPKI FKRFWNAGVV EPVYGPNXSN 1560

QDKILLALSV CEYSVDLFMH DWQGGVPLEI FICDNDPDVA DMRRSSFLAR HLAYLCSLAE 1620

ISRDGPRLES MNSLERLESL KSYLELTFLD DPVLRYSQLT GLVIKVFPST LTYIRKSSIK 1680

VLRTRGIGVP EVLEDWDPEA DNALLDGIAA EIQQNIPLGH QTRAPFWGLR VSKSQVLRLR 1740
 
GYKEITRGEI GRSGVGLTLP FDGRYLSHQL RLFGINSTSC LKALELTYLL SPLVDXDKDR 1800

LYLGEGAGAM LSCYDATLGP CINYYNSGVY SCDVNGQREL NIYPAEVALV GKKLNNVTSL 1860

GQRVKVLFNG NPGSTWIGND ECEALIWNEL QNSSIGLVHC DMEGGDHKDD QVVLHEHYSV 1920

IRIAYLVGDR DVVLISKIAP RLGTDWTRQL SLYLRYWDEV NLIVLKTSNP ASTEMYLLSR 1980

HPKSDIIEDS KTVLASLLPL SKEDSIKIEK WILIEKAKAH EWVTRELREG SSSSGMLRPY 2040

HQALQTFGFE PNLYKLSRDF LSTMNIADTH NCMIAFNRVL KDTIFEWARI TESDKRLKLT 2100

GKYDLYPVRD SGKLKTISRR LVLSWISLSM STRLVTGSFP DQKFEARLQL GIVSLSSREI 2160

RNLRVITKTL LDRFEDIIHS ITYRFLTKEI KILMKILGAV KMFGARQNEY TTVIDDGSLG 2220

DIEPYDSS 2228
SEQ ID NO: 7: Amino acid sequence of a mutant of the Sendai virus L protein (N1197S/L1558I/K1796E) 
MDGQESSQNP SDILYPECHL NSPIVRGKIA QLHVLLDVNQ PYRLKDDSII NITKHKIRNG 60

GLSPRQIKIR SLGKALQRTI KDLDRYTFEP YPTYSQELLR LDIPEICDKI RSVFAVSDRL 120

TRELSSGFQD LWLNIFKQLG NIEGREGYDP LQDIGTIPEI TDKYSRNRWY RPFLTWFSIK 180

YDMRWMQKTR PGGPLDTSNS HNLLECKSYT LVTYGDLVMI LNKLTLTGYI LTPELVLMYC 240

DVVEGRWNMS AAGHLDKKSI GITSKGEELW ELVDSLFSSL GEEIYNVIAL LEPLSLALIQ 300

LNDPVIPLRG AFMRHVLTEL QTVLTSRDVY TDAEADTIVE SLLAIFHGTS IDEKAEIFSF 360

FRTFGHPSLE AVTAADKVRA HMYAQKAIKL KTLYECHAVF CTIIINGYRE RHGGQWPPCD 420

FPDHVCLELR NAQGSNTAIS YECAVDNYTS FIGFKFRKFI EPQLDEDLTI YMKDKALSPR 480

KEAWDSVYPD SNLYYKAPES EETRRLIEVF INDENFNPEE IINYVESGDW LKDEEFNISY 540

SLKEKEIKQE GRLFAKMTYK MRAVQVLAET LLAKGIGELF SENGMVKGEI DLLKRLTTLS 600

VSGVPRTDSV YNNSKSSEKR NEGMENKNSG GYWDEKKRSR HEFKATDSST DGYETLSCFL 660

TTDLKKYCLN WRFESTALFG QRCNEIFGFK TFFNWMHPVL ERCTIYVGDP YCPVADRMHR 720

QLQDHADSGI FIHNPRGGIE GYCQKLWTLI SISAIHLAAV RVGVRVSAMV QGDNQAIAVT 780

SRVPVAQTYK QKKNHVYEEI TKYFGALRHV MFDVGHELKL NETIISSKMF VYSKRIYYDG 840

KILPQCLKAL TKCVFWSETL VDENRSACSN ISTSIAKAIE NGYSPILGYC IALYKTCQQV 900

CISLGMTINP TISPTVRDQY FKGKNWLRCA VLIPANVGGF NYMSTSRCFV RNIGDPAVAA 960

LADLKRFIRA DLLDKQVLYR VMNQEPGDSS FLDWASDPYS CNLPHSQSIT TIIKNITARS 1020
 
VLQESPNPLL SGLFTETSGE EDLNLASFLM DRKVILPRVA HEILGNSLTG VREAIAGMLD 1080

TTKSLVRASV RKGGLSYGIL RRLVNYDLLQ YETLTRTLRK PVKDNIEYEY MCSVELAVGL 1140

RQKMWIHLTY GRPIHGLETP DPLELLRGIF IEGSEVCKLC RSEGADPIYT WFYLPDSIDL 1200

DTLTNGCPAI RIPYFGSATD ERSEAQLGYV RNLSKPAKAA IRIAMVYTWA YGTDEISWME 1260

AALIAQTRAN LSLENLKLLT PVSTSTNLSH RLKDTATQMK FSSATLVRAS RFITISNDNM 1320

ALKEAGESKD TNLVYQQIML TGLSLFEFNM RYKKGSLGKP LILHLHLNNG CCIMESPQEA 1380

NIPPRSTLDL EITQENNKLI YDPDPLKDVD LELFSKVRDV VHTVDMTYWS DDEVIRATSI 1440

CTAMTIADTM SQLDRDNLKE MIALVNDDDV NSLITEFMVI DVPLFCSTFG GILVNQFAYS 1500

LYGLNIRGRE EIWGHVVRIL KDTSHAVLKV LSNALSHPKI FKRFWNAGVV EPVYGPNISN 1560

QDKILLALSV CEYSVDLFMH DWQGGVPLEI FICDNDPDVA DMRRSSFLAR HLAYLCSLAE 1620

ISRDGPRLES MNSLERLESL KSYLELTFLD DPVLRYSQLT GLVIKVFPST LTYIRKSSIK 1680

VLRTRGIGVP EVLEDWDPEA DNALLDGIAA EIQQNIPLGH QTRAPFWGLR VSKSQVLRLR 1740

GYKEITRGEI GRSGVGLTLP FDGRYLSHQL RLFGINSTSC LKALELTYLL SPLVDEDKDR 1800

LYLGEGAGAM LSCYDATLGP CINYYNSGVY SCDVNGQREL NIYPAEVALV GKKLNNVTSL 1860

GQRVKVLFNG NPGSTWIGND ECEALIWNEL QNSSIGLVHC DMEGGDHKDD QVVLHEHYSV 1920

IRIAYLVGDR DVVLISKIAP RLGTDWTRQL SLYLRYWDEV NLIVLKTSNP ASTEMYLLSR 1980

HPKSDIIEDS KTVLASLLPL SKEDSIKIEK WILIEKAKAH EWVTRELREG SSSSGMLRPY 2040

HQALQTFGFE PNLYKLSRDF LSTMNIADTH NCMIAFNRVL KDTIFEWARI TESDKRLKLT 2100

GKYDLYPVRD SGKLKTISRR LVLSWISLSM STRLVTGSFP DQKFEARLQL GIVSLSSREI 2160

RNLRVITKTL LDRFEDIIHS ITYRFLTKEI KILMKILGAV KMFGARQNEY TTVIDDGSLG 2220

DIEPYDSS 2228
SEQ ID NO: 8: Example of a mutant of the Sendai virus P protein 
MDQDAFILKE DSEVEREAPG GRESLSDVIG FLDAVLSSEP TDIGGDRSWL HNTINTPQGP 60

GSAHRAKSEG EGEVSTPSTQ DNRSGEESRV SGRTSKPEAE AHAGNLDKQN IHRAFGGRTG 120

TNSVSQDLGD GGDSGILENP PNERGYPRSG IEDENREMAA HPDKRGEDQA EGLPEEVRGS 180

TSLPDEGEGG ASNNGRSMEP GSSHSARVTG VLVIPSPELE EAVLRRNKRR PTNSGSKPLT 240

PATVPGTRSP PLNRYNSTGS PPGKPPSTQD EHINSGDTPA VRVKDRKPPI GTRSVSDCPA 300
 
NGRPIHPGLE TDSTKKGIGE NTSSMKEMAT LLTSLGVIQS AQEFESSRDA SYVFARRALK 360

SANYAEMTFN VCGLILSAEK SSARKVDENK QLLKQIQESV ESFRDIYKRF SEYQKEQNSL 420

LMSNLSTLHI ITAAGGATDN TDSLTRSPSV FAKSKENKTK ATRFDPSMET LEDMKYKPDL 480

IREDEFRDEI RNPVYQERDT EPRASNASRL YPSKEKPTMH SLRLVIESSP LSRAEKVAYV 540

KSLSKCKTDQ EVKAVMELVE EDIESLTN 568
SEQ ID NO: 9: Example of a mutant of the Sendai virus P protein 
MDQDAFILKE DSEVEREAPG GRESLSDVIG FLDAVLSSEP TDIGGDRSWL HNTINTPQGP 60

GSAHRAKSEG EGEVSTPSTQ DNRSGEESRV SGRTSKPEAE AHAGNLDKQN IHRAFGGRTG 120

TNSVSQDLGD GGDSGILENP PNERGYPRSG IEDENREMAA HPDKRGEDQA EGLPEEVRGS 180

TSLPDEGEGG ASNNGRSMEP GSSHSARVTG VLVIPSPELE EAVLRRNKRR PTNSGSKPLT 240

PATVPGTRSP PLNRYNSTGS PPGKPPSTQD EHINSGDTPA VRVKDRKPPI GTRSVSDCPA 300

NGRPIHPGLE TDSTKKGIGE NTSSMKEMAT LLTSLGVIQS AQEFESSRDA SYVFARRALK 360

SANYAEMTFN VCGLILSAEK SSARKVDENK QLLKQIQESV ESFRDIYKRF SEYQKEQNSL 420

LMSNLSTLHI ITSSGGSTDN TDSLTRSPSV FAKSKENKTK ATRFDPSMET LEDMKYKPDL 480

IREDEFRDEI RNPVYQERDT EPRASNASRL YPSKEKPTMH SLRLVIESSP LSRAEKVAYV 540

KSLSKCKTDQ EVKAVMELVE EDIESLTN 568
SEQ ID NO: 10: Example of a mutant of the Sendai virus P protein 
MDQDAFILKE DSEVEREAPG GRESLSDVIG FLDAVLSSEP TDIGGDRSWL HNTINTPQGP 60

GSAHRAKSEG EGEVSTPSTQ DNRSGEESRV SGRTSKPEAE AHAGNLDKQN IHRAFGGRTG 120

TNSVSQDLGD GGDSGILENP PNERGYPRSG IEDENREMAA HPDKRGEDQA EGLPEEVRGS 180

TSLPDEGEGG ASNNGRSMEP GSSHSARVTG VLVIPSPELE EAVLRRNKRR PTNSGSKPLT 240

PATVPGTRSP PLNRYNSTGS PPGKPPSTQD EHINSGDTPA VRVKDRKPPI GTRSVSDCPA 300

NGRPIHPGLE TDSTKKGIGE NTSSMKEMAT LLTSLGVIQS AQEFESSRDA SYVFARRALK 360

SANYAEMTFN VCGLILSAEK SSARKVDENK QLLKQIQESV ESFRDIYKRF SEYQKEQNSL 420

LMSNLSTLHI ITTTGGTTDN TDSLTRSPSV FAKSKENKTK ATRFDPSMET LEDMKYKPDL 480

IREDEFRDEI RNPVYQERDT EPRASNASRL YPSKEKPTMH SLRLVIESSP LSRAEKVAYV 540
 KSLSKCKTDQ EVKAVMELVE EDIESLTN 568
SEQ ID NO: 11: Example of a mutant of the Sendai virus P protein 
MDQDAFILKE DSEVEREAPG GRESLSDVIG FLDAVLSSEP TDIGGDRSWL HNTINTPQGP 60

GSAHRAKSEG EGEVSTPSTQ DNRSGEESRV SGRTSKPEAE AHAGNLDKQN IHRAFGGRTG 120

TNSVSQDLGD GGDSGILENP PNERGYPRSG IEDENREMAA HPDKRGEDQA EGLPEEVRGS 180

TSLPDEGEGG ASNNGRSMEP GSSHSARVTG VLVIPSPELE EAVLRRNKRR PTNSGSKPLT 240

PATVPGTRSP PLNRYNSTGS PPGKPPSTQD EHINSGDTPA VRVKDRKPPI GTRSVSDCPA 300

NGRPIHPGLE TDSTKKGIGE NTSSMKEMAT LLTSLGVIQS AQEFESSRDA SYVFARRALK 360

SANYAEMTFN VCGLILSAEK SSARKVDENK QLLKQIQESV ESFRDIYKRF SEYQKEQNSL 420

LMSNLSTLHI ITGGGGGTDN TDSLTRSPSV FAKSKENKTK ATRFDPSMET LEDMKYKPDL 480

IREDEFRDEI RNPVYQERDT EPRASNASRL YPSKEKPTMH SLRLVIESSP LSRAEKVAYV 540

KSLSKCKTDQ EVKAVMELVE EDIESLTN 568
SEQ ID NO: 12: Example of a mutant of the Sendai virus P protein 
MDQDAFILKE DSEVEREAPG GRESLSDVIG FLDAVLSSEP TDIGGDRSWL HNTINTPQGP 60

GSAHRAKSEG EGEVSTPSTQ DNRSGEESRV SGRTSKPEAE AHAGNLDKQN IHRAFGGRTG 120

TNSVSQDLGD GGDSGILENP PNERGYPRSG IEDENREMAA HPDKRGEDQA EGLPEEVRGS 180

TSLPDEGEGG ASNNGRSMEP GSSHSARVTG VLVIPSPELE EAVLRRNKRR PTNSGSKPLT 240

PATVPGTRSP PLNRYNSTGS PPGKPPSTQD EHINSGDTPA VRVKDRKPPI GTRSVSDCPA 300

NGRPIHPGLE TDSTKKGIGE NTSSMKEMAT LLTSLGVIQS AQEFESSRDA SYVFARRALK 360

SANYAEMTFN VCGLILSAEK SSARKVDENK QLLKQIQESV ESFRDIYKRF SEYQKEQNSL 420

LMSNLSTLHI ITSSGGSTDN TDSLTRSPSV FAKSKENKTK ATRFDPSMET LEDMKYKPDL 480

IREDEFRDEI RNPVYQERDT EPRASNASRL FPSKEKPTMH SLRLVIESSP LSRAEKVAYV 540

KSLSKCKTDQ EVKAVMELVE EDIESLTN 568
SEQ ID NO: 13: Example of a mutant of the Sendai virus P protein MDQDAFILKE DSEVEREAPG GRESLSDVIG FLDAVLSSEP TDIGGDRSWL HNTINTPQGP 60 
GSAHRAKSEG EGEVSTPSTQ DNRSGEESRV SGRTSKPEAE AHAGNLDKQN IHRAFGGRTG 120

TNSVSQDLGD GGDSGILENP PNERGYPRSG IEDENREMAA HPDKRGEDQA EGLPEEVRGS 180

TSLPDEGEGG ASNNGRSMEP GSSHSARVTG VLVIPSPELE EAVLRRNKRR PTNSGSKPLT 240

PATVPGTRSP PLNRYNSTGS PPGKPPSTQD EHINSGDTPA VRVKDRKPPI GTRSVSDCPA 300

NGRPIHPGLE TDSTKKGIGE NTSSMKEMAT LLTSLGVIQS AQEFESSRDA SYVFARRALK 360

SANYAEMTFN VCGLILSAEK SSARKVDENK QLLKQIQESV ESFRDIYKRF SEYQKEQNSL 420

LMSNLSTLHI ITSAGGATDN TDSLTRSPSV FAKSKENKTK ATRFDPSMET LEDMKYKPDL 480

IREDEFRDEI RNPVYQERDT EPRASNASRL FPSKEKPTMH SLRLVIESSP LSRAEKVAYV 540

KSLSKCKTDQ EVKAVMELVE EDIESLTN 568
SEQ ID NO: 14: Example of a mutant of the Sendai virus P protein 
MDQDAFILKE DSEVEREAPG GRESLSDVIG FLDAVLSSEP TDIGGDRSWL HNTINTPQGP 60

GSAHRAKSEG EGEVSTPSTQ DNRSGEESRV SGRTSKPEAE AHAGNLDKQN IHRAFGGRTG 120

TNSVSQDLGD GGDSGILENP PNERGYPRSG IEDENREMAA HPDKRGEDQA EGLPEEVRGS 180

TSLPDEGEGG ASNNGRSMEP GSSHSARVTG VLVIPSPELE EAVLRRNKRR PTNSGSKPLT 240

PATVPGTRSP PLNRYNSTGS PPGKPPSTQD EHINSGDTPA VRVKDRKPPI GTRSVSDCPA 300

NGRPIHPGLE TDSTKKGIGE NTSSMKEMAT LLTSLGVIQS AQEFESSRDA SYVFARRALK 360

SANYAEMTFN VCGLILSAEK SSARKVDENK QLLKQIQESV ESFRDIYKRF SEYQKEQNSL 420

LMSNLSTLHI ITSAGGATDN TDSLTRSPSV FAKSKENKTK ATRFDPSMET LEDMKYKPDL 480

IREDEFRDEI RNPVYQERDT EPRASNASRL YPSKEKPTMH SLRLVIESSP LSRAEKVAYV 540

KSLSKCKTDQ EVKAVMELVE EDIESLTN 568
SEQ ID NO: 15: Example of a mutant of the Sendai virus P protein 
MDQDAFILKE DSEVEREAPG GRESLSDVIG FLDAVLSSEP TDIGGDRSWL HNTINTPQGP 60

GSAHRAKSEG EGEVSTPSTQ DNRSGEESRV SGRTSKPEAE AHAGNLDKQN IHRAFGGRTG 120

TNSVSQDLGD GGDSGILENP PNERGYPRSG IEDENREMAA HPDKRGEDQA EGLPEEVRGS 180

TSLPDEGEGG ASNNGRSMEP GSSHSARVTG VLVIPSPELE EAVLRRNKRR PTNSGSKPLT 240

PATVPGTRSP PLNRYNSTGS PPGKPPSTQD EHINSGDTPA VRVKDRKPPI GTRSVSDCPA 300
 
NGRPIHPGLE TDSTKKGIGE NTSSMKEMAT LLTSLGVIQS AQEFESSRDA SYVFARRALK 360

SANYAEMTFN VCGLILSAEK SSARKVDENK QLLKQIQESV ESFRDIYKRF SEYQKEQNSL 420

LMSNLSTLHI ITASGGATDN TDSLTRSPSV FAKSKENKTK ATRFDPSMET LEDMKYKPDL 480

IREDEFRDEI RNPVYQERDT EPRASNASRL YPSKEKPTMH SLRLVIESSP LSRAEKVAYV 540

KSLSKCKTDQ EVKAVMELVE EDIESLTN 568
SEQ ID NO: 16: Example of a mutant of the Sendai virus P protein 
MDQDAFILKE DSEVEREAPG GRESLSDVIG FLDAVLSSEP TDIGGDRSWL HNTINTPQGP 60

GSAHRAKSEG EGEVSTPSTQ DNRSGEESRV SGRTSKPEAE AHAGNLDKQN IHRAFGGRTG 120

TNSVSQDLGD GGDSGILENP PNERGYPRSG IEDENREMAA HPDKRGEDQA EGLPEEVRGS 180

TSLPDEGEGG ASNNGRSMEP GSSHSARVTG VLVIPSPELE EAVLRRNKRR PTNSGSKPLT 240

PATVPGTRSP PLNRYNSTGS PPGKPPSTQD EHINSGDTPA VRVKDRKPPI GTRSVSDCPA 300

NGRPIHPGLE TDSTKKGIGE NTSSMKEMAT LLTSLGVIQS AQEFESSRDA SYVFARRALK 360

SANYAEMTFN VCGLILSAEK SSARKVDENK QLLKQIQESV ESFRDIYKRF SEYQKEQNSL 420

LMSNLSTLHI ITAAGGSTDN TDSLTRSPSV FAKSKENKTK ATRFDPSMET LEDMKYKPDL 480

IREDEFRDEI RNPVYQERDT EPRASNASRL YPSKEKPTMH SLRLVIESSP LSRAEKVAYV 540

KSLSKCKTDQ EVKAVMELVE EDIESLTN 568
SEQ ID NO: 17: Example of a mutant of the Sendai virus L protein 
MDGQESSQNP SDILYPECHL NSPIVRGKIA QLHVLLDVNQ PYRLKDDSII NITKHKIRNG 60

GLSPRQIKIR SLGKALQRTI KDLDRYTFEP YPTYSQELLR LDIPEICDKI RSVFAVSDRL 120

TRELSSGFQD LWLNIFKQLG NIEGREGYDP LQDIGTIPEI TDKYSRNRWY RPFLTWFSIK 180

YDMRWMQKTR PGGPLDTSNS HNLLECKSYT LVTYGDLVMI LNKLTLTGYI LTPELVLMYC 240

DVVEGRWNMS AAGHLDKKSI GITSKGEELW ELVDSLFSSL GEEIYNVIAL LEPLSLALIQ 300

LNDPVIPLRG AFMRHVLTEL QTVLTSRDVY TDAEADTIVE SLLAIFHGTS IDEKAEIFSF 360

FRTFGHPSLE AVTAADKVRA HMYAQKAIKL KTLYECHAVF CTIIINGYRE RHGGQWPPCD 420

FPDHVCLELR NAQGSNTAIS YECAVDNYTS FIGFKFRKFI EPQLDEDLTI YMKDKALSPR 480

KEAWDSVYPD SNLYYKAPES EETRRLIEVF INDENFNPEE IINYVESGDW LKDEEFNISY 540
 
SLKEKEIKQE GRLFAKMTYK MRAVQVLAET LLAKGIGELF SENGMVKGEI DLLKRLTTLS 600

VSGVPRTDSV YNNSKSSEKR NEGMENKNSG GYWDEKKRSR HEFKATDSST DGYETLSCFL 660

TTDLKKYCLN WRFESTALFG QRCNEIFGFK TFFNWMHPVL ERCTIYVGDP YCPVADRMHR 720

QLQDHADSGI FIHNPRGGIE GYCQKLWTLI SISAIHLAAV RVGVRVSAMV QGDNQAIAVT 780

SRVPVAQTYK QKKNHVYEEI TKYFGALRHV MFDVGHELKL NETIISSKMF VYSKRIYYDG 840

KILPQCLKAL TKCVFWSETL VDENRSACSN ISTSIAKAIE NGYSPILGYC IALYKTCQQV 900

CISLGMTINP TISPTVRDQY FKGKNWLRCA VLIPANVGGF NYMSTSRCFV RNIGDPAVAA 960

LADLKRFIRA DLLDKQVLYR VMNQEPGDSS FLDWASDPYS CNLPHSQSIT TIIKNITARS 1020

VLQESPNPLL SGLFTETSGE EDLNLASFLM DRKVILPRVA HEILGNSLTG VREAIAGMLD 1080

TTKSLVRASV RKGGLSYGIL RRLVNYDLLQ YETLTRTLRK PVKDNIEYEY MCSVELAVGL 1140

RQKMWIHLTY GRPIHGLETP DPLELLRGIF IEGSEVCKLC RSEGADPIYT WFYLPDSIDL 1200

DTLTNGCPAI RIPYFGSATD ERSEAQLGYV RNLSKPAKAA IRIAMVYTWA YGTDEISWME 1260

AALIAQTRAN LSLENLKLLT PVSTSTNLSH RLKDTATQMK FSSATLVRAS RFITISNDNM 1320

ALKEAGESKD TNLVYQQIML TGLSLFEFNM RYKKGSLGKP CILHLHLNNG CCIMESPQEA 1380

NIPPRSTLDL EITQENNKLI YDPDPLKDVD LELFSKVRDV VHTVDMTYWS DDEVIRATSI 1440

CTAMTIADTM SQLDRDNLKE MIALVNDDDV NSLITEFMVI DVPLFCSTFG GILVNQFAYS 1500

LYGLNIRGRE EIWGHVVRIL KDTSHAVLKV LSNALSHPKI FKRFWNAGVV EPVYGPNISN 1560

QDKILLALSV CEYSVDLFMH DWQGGVPLEI FICDNDPDVA DMRRSSFLAR HLAYLCSLAE 1620

ISRDGPRLES MNSLERLESL KSYLELTFLD DPVLRYSQLT GLVIKVFPST LTYIRKSSIK 1680

VLRTRGIGVP EVLEDWDPEA DNALLDGIAA EIQQNIPLGH QTRAPFWGLR VSKSQVLRLR 1740

GYKEITRGEI GRSGVGLTLP FDGRYLSHQL RLFGINSTSC LKALELTYLL SPLVDEDKDR 1800

LYLGEGAGAM LSCYDATLGP CINYYNSGVY SCDVNGQREL NIYPAEVALV GKKLNNVTSL 1860

GQRVKVLFNG NPGSTWIGND ECEALIWNEL QNSSIGLVHC DMEGGDHKDD QVVLHEHYSV 1920

IRIAYLVGDR DVVLISKIAP RLGTDWTRQL SLYLRYWDEV NLIVLKTSNP ASTEMYLLSR 1980

HPKSDIIEDS KTVLASLLPL SKEDSIKIEK WILIEKAKAH EWVTRELREG SSSSGMLRPY 2040

HQALQTFGFE PNLYKLSRDF LSTMNIADTH NCMIAFNRVL KDTIFEWARI TESDKRLKLT 2100

GKYDLYPVRD SGKLKTISRR LVLSWISLSM STRLVTGSFP DQKFEARLQL GIVSLSSREI 2160

RNLRVITKTL LDRFEDIIHS ITYRFLTKEI KILMKILGAV KMFGARQNEY TTVIDDGSLG 2220
 DIEPYDSS 2228
SEQ ID NO: 18: Example of a DD tag 
GVQVETISPG DGRTFPKRGQ TCVVHYTGML EDGKKVDSSR DRNKPFKFML GKQEVIRGWE 60

EGVAQMSVGQ RAKLTISPDY AYGATGHPGI IPPHATLVFD VELLKP 106
SEQ ID NO: 19: Example of a DDm tag 
GVQIETISPG DGRTFPKKGQ TCVVHYTGML EDGKKLDSSR DRNKPFKFMI GKQEVIKGFE 60

EGAAQMSLGQ RAKLTISPDY AYGATGHPGV IPPHATLIFD VELLRPE 107
SEQ ID NO: 20: Mutant of the M protein 
MADIYRFPKF SYEDNGTVEP LPLRTGPDKK AIPHIRIVKV GDPPKHGVRY LDLLLLGFFE 60

TPKQTTNLES VSDLTEPTSY SICGSGSLPI GVAKYYGTDQ ELLKACTDLR ITVRRAVRAG 120

EMIVYMVDSI GAPLLPWSGR LRQGMIFNAN KVALAPQCLP VDKDIRLRVV FVNGTSLGAI 180

TITKIPKTLA DLALPNSISV NLLVTLKTGI STEQKGVLPV LDDQGEKKLN FMVHLGLIRR 240

KVGKIYSVEY CKSKIERMRL IFSLGLIGGI SFHVQVNGTL SKTFMSQLAW KRAVCFPLMD 300

VNPHMNMVIW AASVEITGVD AVFQPAIPRD FRYYPNVVAK NIGRIRKL 348
SEQ ID NO: 21: Mutant of the HN protein 
MDGDRGKRDS YWSTSPSGST TKPASGWERS SKADTWLLIL SFTQWALSIA TVIICIIISA 60

RQGYSMKEYS MTVEALNMSS REVKESLTSL IRQEVIARAV NIQSSVQTGI PVLLNKNSRD 120

VIQMIDKSCS RQELTQHCES TIAVHHADGI APLEPHSFWR CPVGEPYLSS DPEISLLPGP 180

SLLSGSTTIS GCVRLPSLSI GEAIYAYSSN LITQGCADIG KSYQVLQLGY ISLNSDMFPD 240

LNPVVSHTYD INDNRKSCSV VTTKTRGYQL CSMPTVDERT DYSSDGIEDL VLDVLDLKGR 300

TKSHRYRNSE VDLDHPFSAL YPSVGNGIAT EGSLIFLGYG GLTTPLQGDT KCRTQGCQQV 360

SQDTCNEALK ITWLGGKQVV SVIIQVNDYL SERPKIRVTT IPITQNYLGA EGRLLKLGDR 420

VYIYTRSSGW HSQLQIGVLD VSHPLTINWT PHEALSRPGN EECNWYNKCP KECISGVYTD 480

AYPLSPDAAN VATVTLYANT SRVNPTIMYS NTTNIINMLR IKDVQLEAAY TTTSCITHFG 540

KGYCFHIIEI NQKSLNTLQP MLFKTSIPKL CKAES 575
SEQ ID NO: 22: Example of a bovine FKBP12 domain corresponding to the DD domain of human FKBP12 
MGVQVETISP GDGRTFPKRG QTCVVHYTGM LEDGKKFDSS RDRNKPFKFV LGKQEVIRGW 60

EEGVAQMSVG QRAKLTISPD YAYGATGHPG IIPPNATLIF DVELLKLE 108
SEQ ID NO: 23: Example of a rhesus monkey FKBP12 domain corresponding to the DD domain of human FKBP12 
MGVEIETISP GDGRTFPKKG QTCVVHYTGM LQNGKKFDSS RDRNKPFKFR IGKQEVIKGF 60

EEGAAQMSLG QRAKLTCTPD VAYGATGHPG VIPPNATLIF DVELLNLE 108
SEQ ID NO: 24: Example of human FKBP12 
MGVQVETISP GDGRTFPKRG QTCVVHYTGM LEDGKKFDSS RDRNKPFKFM LGKQEVIRGW 60

EEGVAQMSVG QRAKLTISPD YAYGATGHPG IIPPHATLVF DVELLKLE 108
SEQ ID NO: 25: Example of a mouse FKBP12 domain corresponding to the DD domain of human FKBP12 
MGVEIETISP GDGRTFPKKG QICVVHYTGM LQNGKKFDSS RDRNKPFKFR IGKQEVIKGF 60

EEGTAQMSLG QRAKLTCTPD VAYGATGHPG VIPPNATLIF DVELLSLE 108
SEQ ID NO: 26: Example of a rabbit FKBP12 domain corresponding to the DD domain of human FKBP12 
MGVQVETISP GDGRTFPKRG QTCVVHYTGM LEDGKKFDSS RDRNKPFKFM LGKQEVIRGW 60

EEGVAQMSVG QRAKLTISPD YAYGATGHPG IIPPHATLVF DVELLKLE 108
SEQ ID NO: 27: Example of a rat FKBP12 domain corresponding to the DD domain of human FKBP12 
MGVEIETISP GDGRTFPKKG QICVVHYTGM LQNGKKFDSS RDRNKPFKFR IGKQEVIKGF 60

EEGAAQMSLG QRAKLTCTPD VAYGATGHPG VIPPNATLIF DVELLNLE 108
SEQ ID NO: 28: Example of a DDrs tag 
GVQVETISPG DGRTFPRRGQ TCVVHYTGML EDGRRVDSSR DRNRPFRFML GRQEVIRGWE 60

EGVSQMSVGQ RSRLTISPDY SYGSTGHPGI IPPHSTLVFD VELLRPE 107
SEQ ID NO: 29: Brd4BD2(L387A) (bromotag) 
VKDVPDSQQH PAPEKSSKVS EQLKCCSGIL KEMFAKKHAA YAWPFYKPVD VEALGAHDYC 60

DIIKHPMDMS TIKSKLEARE YRDAQEFGAD VRLMFSNCYK YNPPDHEVVA MARKLQDVFE 120
 MRFAKMPDE
SEQ ID NO: 30: P2m3Syb 
MDQDAFILKE DSEVEREAPG GRESLSDVIG FLDAVLSSEP TDIGGDRSWL HNTINTPQGP 60

GSAHRAKSEG EGEVSTPSTQ DNRSGEESRV SGRTSKPEAE AHAGNLDKQN IHRAFGGRTG 120

TNSVSQDLGD GGDSGILENP PNERGYPRSG IEDENREMAA HPDKRGEDQA EGLPEEVRGS 180

TSLPDEGEGG ASNNGRSMEP GSSHSARVTG VLVIPSPELE EAVLRRNKRR PTNSGSKPLT 240

PATVPGTRSP PLNRYNSTGS PPGKPPSTQD EHINSGDTPA VRVKDRKPPI GTRSVSDCPA 300

NGRPIHPGLE TDSTKKGIGE NTSSMKEMAT LLTSLGVIQS AQEFESSRDA SYVFARRALK 360

SANYAEMTFN VCGLILSAEK SSARKVDENK QLLKQIQESV ESFRDIYKRF SEYQKEQNSL 420

LMSNLSTLHI ITSSGGSTDN TDSLTRSPSV FAKSKENKTK ATRFDPSMET LEDMKYKPDL 480

IREDEFRDEI RNPVYQERDT EPRASNASRL YPSKEKPTMH SLRLVIESSP LSRAEKVAYV 540

KSLSKCKTDQ EVKAVMELVE EDIESLTNKL GSGSVKDVPD SQQHPAPEKS SKVSEQLKCC 600

SGILKEMFAK KHAAYAWPFY KPVDVEALGA HDYCDIIKHP MDMSTIKSKL EAREYRDAQE 660

FGADVRLMFS NCYKYNPPDH EVVAMARKLQ DVFEMRFAKM PDE
SEQ ID NO: 31: P2m2S434Tyb 
MDQDAFILKE DSEVEREAPG GRESLSDVIG FLDAVLSSEP TDIGGDRSWL HNTINTPQGP 60

GSAHRAKSEG EGEVSTPSTQ DNRSGEESRV SGRTSKPEAE AHAGNLDKQN IHRAFGGRTG 120

TNSVSQDLGD GGDSGILENP PNERGYPRSG IEDENREMAA HPDKRGEDQA EGLPEEVRGS 180

TSLPDEGEGG ASNNGRSMEP GSSHSARVTG VLVIPSPELE EAVLRRNKRR PTNSGSKPLT 240

PATVPGTRSP PLNRYNSTGS PPGKPPSTQD EHINSGDTPA VRVKDRKPPI GTRSVSDCPA 300

NGRPIHPGLE TDSTKKGIGE NTSSMKEMAT LLTSLGVIQS AQEFESSRDA SYVFARRALK 360

SANYAEMTFN VCGLILSAEK SSARKVDENK QLLKQIQESV ESFRDIYKRF SEYQKEQNSL 420

LMSNLSTLHI ITSTGGSTDN TDSLTRSPSV FAKSKENKTK ATRFDPSMET LEDMKYKPDL 480

IREDEFRDEI RNPVYQERDT EPRASNASRL YPSKEKPTMH SLRLVIESSP LSRAEKVAYV 540

KSLSKCKTDQ EVKAVMELVE EDIESLTNKL GSGSVKDVPD SQQHPAPEKS SKVSEQLKCC 600

SGILKEMFAK KHAAYAWPFY KPVDVEALGA HDYCDIIKHP MDMSTIKSKL EAREYRDAQE 660
 FCADVRLMFS NCYKYNPPDH EVVAMARKLQ DVFEMRFAKM PDE
SEQ ID NO: 32: P2m3Tyb 
MDQDAFILKE DSEVEREAPG GRESLSDVIG FLDAVLSSEP TDIGGDRSWL HNTINTPQGP 60

GSAHRAKSEG EGEVSTPSTQ DNRSGEESRV SGRTSKPEAE AHAGNLDKQN IHRAFGGRTG 120

TNSVSQDLGD GGDSGILENP PNERGYPRSG IEDENREMAA HPDKRGEDQA EGLPEEVRGS 180

TSLPDEGEGG ASNNGRSMEP GSSHSARVTG VLVIPSPELE EAVLRRNKRR PTNSGSKPLT 240

PATVPGTRSP PLNRYNSTGS PPGKPPSTQD EHINSGDTPA VRVKDRKPPI GTRSVSDCPA 300

NGRPIHPGLE TDSTKKGIGE NTSSMKEMAT LLTSLGVIQS AQEFESSRDA SYVFARRALK 360

SANYAEMTFN VCGLILSAEK SSARKVDENK QLLKQIQESV ESFRDIYKRF SEYQKEQNSL 420

LMSNLSTLHI ITTTGGTTDN TDSLTRSPSV FAKSKENKTK ATRFDPSMET LEDMKYKPDL 480

IREDEFRDEI RNPVYQERDT EPRASNASRL YPSKEKPTMH SLRLVIESSP LSRAEKVAYV 540

KSLSKCKTDQ EVKAVMELVE EDIESLTNKL GSGSVKDVPD SQQHPAPEKS SKVSEQLKCC 600

SGILKEMFAK KHAAYAWPFY KPVDVEALGA HDYCDIIKHP MDMSTIKSKL EAREYRDAQE 660

FGADVRLMFS NCYKYNPPDH EVVAMARKLQ DVFEMRFAKM PDE
SEQ ID NO: 33: EmGFPb

MVSKGEELFT GVVPILVELD GDVNGHKFSV SGEGEGDATY GKLTLKFICT TGKLPVPWPT 60

LVTTLTYGVQ CFARYPDHMK QHDFFKSAMP EGYVQERTIF FKDDGNYKTR AEVKFEGDTL 120

VNRIELKGID FKEDGNILGH KLEYNYNSHK VYITADKQKN GIKVNFKTRH NIEDGSVQLA 180

DHYQQNTPIG DGPVLLPDNH YLSTQSALSK DPNEKRDHMV LLEFVTAAGI TLGMDELYKL 240

GSGSVKDVPD SQQHPAPEKS SKVSEQLKCC SGILKEMFAK KHAAYAWPFY KPVDVEALGA 300

HDYCDIIKHP MDMSTIKSKL EAREYRDAQE FGADVRLMFS NCYKYNPPDH EVVAMARKLQ 360

DVFEMRFAKM PDE

## Claims

1. An RNA genome of a negative-strand RNA virus or virus vector, wherein at least one of the viral proteins encoded by the genome (preferably the P protein or a temperature-sensitive P protein) is in the form of a fusion protein with a bromotag.

2. The RNA genome of the negative-strand RNA virus or viral vector according to Claim 1,
wherein the temperature-sensitive P protein encoded by the virus genome has a substitution mutation(s) in an amino acid(s) of the P protein corresponding to one or more or all of D433, R434, and K437, the substitution mutation(s) comprising a mutation to any of G, T, and S, and optionally further has a substitution mutation in an amino acid of the P protein corresponding to L511, the substitution mutation in L511 being a mutation to Y or F, or
wherein the P protein encoded by the virus genome has substitution mutations in the amino acids of the P protein corresponding to all of D433, R434, and K437, and each of the substitution mutations comprises a mutation to any of G, T, and S.

3. The RNA genome of a negative-strand RNA virus or virus vector according to claim 2, wherein each of the substitution mutations is a mutation to T or S.

4. The RNA genome of a negative-strand RNA virus or virus vector according to claim 3, wherein each of the substitution mutations is a mutation to T.

5. The RNA genome of a negative-strand RNA virus or virus vector according to claim 3, wherein each of the substitution mutations is a mutation to S.

6. The RNA genome of a negative-strand RNA virus or virus vector according to any one of claims 1 to 3, wherein the temperature-sensitive P protein encoded by the virus genome:
(1S) has amino acid mutations corresponding to D433S, R434A, K437A and L511F;
(2S) has amino acid mutations corresponding to D433S, R434A, K437A and L511Y;
(3S) has amino acid mutations corresponding to D433A, R434S, K437A and L511F;
(4S) has amino acid mutations corresponding to D433A, R434S, K437A and L511Y;
(5S) has amino acid mutations corresponding to D433A, R434A, K437S and L511F;
(6S) has amino acid mutations corresponding to D433A, R434A, K437S and L511Y;
(7S) has amino acid mutations corresponding to D433S, R434S, K437A and L511F;
(8S) has amino acid mutations corresponding to D433S, R434S, K437A and L511Y;
(9S) has amino acid mutations corresponding to D433S, R434A, K437S and L511F;
(10S) has amino acid mutations corresponding to D433S, R434A, K437S and L511Y;
(11S) has amino acid mutations corresponding to D433A, R434S, K437S and L511F;
(12S) has amino acid mutations corresponding to D433A, R434S, K437S and L511Y;
(13S) has amino acid mutations corresponding to D433S, R434S, K437S and L511F;
(14S) has amino acid mutations corresponding to D433S, R434S, K437S and L511Y;
(1T) has amino acid mutations corresponding to D433T, R434A, K437A and L511F;
(2T) has amino acid mutations corresponding to D433T, R434A, K437A and L511Y;
(3T) has amino acid mutations corresponding to D433A, R434T, K437A and L511F;
(4T) has amino acid mutations corresponding to D433A, R434T, K437A and L511Y;
(5T) has amino acid mutations corresponding to D433A, R434A, K437T and L511F;
(6T) has amino acid mutations corresponding to D433A, R434A, K437T and L511Y;
(7T) has amino acid mutations corresponding to D433T, R434T, K437A and L511F;
(8T) has amino acid mutations corresponding to D433T, R434T, K437A and L511Y;
(9T) has amino acid mutations corresponding to D433T, R434A, K437T and L511F;
(10T) has amino acid mutations corresponding to D433T, R434A, K437T and L511Y;
(11T) has amino acid mutations corresponding to D433A, R434T, K437T and L511F;
(12T) has amino acid mutations corresponding to D433A, R434T, K437T and L511Y;
(13T) has amino acid mutations corresponding to D433T, R434T, K437T and L511F;
(14T) has amino acid mutations corresponding to D433T, R434T, K437T and L511Y;
(1ST) has amino acid mutations corresponding to D433S, R434T, K437S and L511F; or
(2ST) has amino acid mutations corresponding to D433S, R434T, K437S and L511Y.

7. The RNA genome of a negative-strand RNA virus or virus vector according to any one of claims 1 to 6, wherein a large protein (L protein) encoded on the virus genome has an amino acid mutation(s) corresponding to one or both of L1361C and L1558I.

8. The RNA genome of a negative-strand RNA virus or virus vector according to any one of claims 1 to 7, having at least one or more exogenous gene of interest loaded thereon.

9. The RNA genome of a negative-strand RNA virus or virus vector according to any one of claims 1 to 8, which is a Sendai virus or Sendai virus vector.

10. A nucleic acid encoding the RNA genome of a negative-strand RNA virus or virus vector according to any one of claims 1 to 9.

11. A negative-strand RNA virus or viral vector, wherein at least one of the viral proteins encoded by the genome (preferably the P protein or a temperature-sensitive P protein) is in the form of a fusion protein with a bromotag, and/or at least one of the viral proteins constituting the virus or viral vector (preferably the P protein or a temperature-sensitive P protein) is in the form of a fusion protein with a bromotag, or
wherein the negative-strand RNA virus or viral vector comprising the RNA genome of the negative-strand RNA virus or viral vector according to any one of claims 1 to 9.

12. The negative-strand RNA virus or virus vector according to claim 11, comprising either a P protein weaker in temperature sensitivity than the P protein encoded on the genome or a P protein having no temperature sensitivity.

13. The negative-strand RNA virus or virus vector according to claim 11 or 12, wherein when the L protein encoded on the virus genome is temperature-sensitive, the virus or virus vector comprises an L protein weaker in temperature sensitivity than the L protein encoded on the genome or an L protein having no temperature sensitivity.

14. A composition comprising the negative-strand RNA virus or virus vector according to any one of claims 11 to 13.

15. A method expressing a gene of interest in a cell, a method for producing a cell expressing a gene of interest, or a method for culturing a cell, comprising:
infecting the cell with the negative-strand RNA virus or virus vector according to any one of claims 11 to 13 and then culturing the cell under the first temperature conditions to express the gene of interest in the cell; and
thereafter further culturing the cell under the second temperature conditions, thereby partly or fully eliminating the negative-strand RNA virus or virus vector from the cell.

16. A method for expressing a gene of interest in a cell, a method for producing a cell expressing a gene of interest, or a method for culturing a cell, comprising:
infecting the cell with the negative-strand RNA virus or virus vector according to any one of claims 11 to 13 and then culturing the cell under the first temperature conditions to express the gene of interest in the cell.

17. A method for expressing a gene of interest in a cell, a method for producing a cell expressing a gene of interest, or a method for culturing a cell, comprising:
infecting the cell with the negative-strand RNA virus or virus vector according to any one of claims 11 to 13 and then culturing the cell under the second temperature conditions, to express the gene of interest in the cell while eliminating the virus or virus vector from the cell.

18. A degradation-inducible protein having an amino acid sequence having amino acid mutations corresponding to two or more or all amino acid mutations selected from the group consisting of 4I, 18K, 50I, 57K, 59F, 63A, 68L, 90V, 98I, and 105R and an amino acid mutation corresponding to 36L, relative to the amino acid sequence set forth in SEQ ID NO: 18.

19. The degradation-inducible protein according to claim 18, having amino acid mutations corresponding to all of 4I, 18K, 36L, 50I, 57K, 59F, 63A, 68L, 90V, 98I, and 105R, relative to the amino acid sequence set forth in SEQ ID NO: 18.

20. A degradation-inducible fusion protein comprising a protein of interest and the degradation-inducible protein according to claim 18 or 19.

21. A nucleic acid encoding the protein according to any one of claims 18 to 20.

22. An animal cell comprising: the protein according to any one of claims 18 to 20 and/or the nucleic acid according to claim 21.

23. A negative-strand RNA virus or vector comprising: the protein according to any one of claims 18 to 20 and/or the nucleic acid according to claim 21.

24. A negative-strand RNA virus or vector comprising the nucleic acid according to claim 21.

25. An M protein comprising the amino acid sequence set forth in SEQ ID NO: 19.

26. A nucleic acid encoding the M protein according to claim 25.

27. An HN protein comprising the amino acid sequence set forth in SEQ ID NO: 20.

28. A nucleic acid encoding the HN protein according to claim 27.

29. An animal cell comprising: the M protein according to claim 25 and/or the HN protein according to claim 27.

30. A negative-strand RNA virus or vector comprising: the M protein according to claim 25 and/or the HN protein according to claim 27.

31. An animal cell comprising the nucleic acid according to claim 26 and/or the nucleic acid according to claim 27.

32. A negative-strand RNA virus or vector comprising a genome of a negative-strand RNA virus or vector comprising the nucleic acid according to claim 26 and/or the nucleic acid according to claim 27.

33. A nucleic acid encoding a genome of a negative-strand RNA virus or vector comprising the nucleic acid according to claim 26 and/or the nucleic acid according to claim 27, or a vector comprising the nucleic acid.
